# EUROPEAN PATENT APPLICATION

(11) **EP 2 383 336 A1**
(43) Date of publication of application: **02.11.2011**
(21) Application number: 10733458.3
(22) Date of filing: 19.01.2010
(51) Int. Cl.: C12N 15/09, A61K 38/00, A61P 9/00, A61P 13/12, A61P 19/02, A61P 29/00, A61P 37/02, C07K 7/08, C07K 19/00, C12N 1/15, C12N 1/19, C12N 1/21, C12N 5/10, C12P 21/02, G01N 33/53

(54) **PEPTIDE CAPABLE OF BINDING TO IMMUNOGLOBULIN**

(30) Priority: 23.01.2009 JP 2009012972
(71) Applicant: MMT CO., LTD, Osaka-shi, Osaka 540-0008 (JP)
(72) Inventor: MASAKI, Osamu, Osaka-shi Osaka 540-0008 (JP)
(74) Representative: Schnappauf, Georg
(86) International application number: PCT/JP2010/050539
(87) International publication number: WO 2010/084851

(57) **Abstract**

The present invention provides a peptide capable of binding to an immunoglobulin, a fusion protein with the peptide, nucleic acids coding for the peptide and for the fusion protein, methods for producing the peptide and the fusion protein, and a composition and means for binding an immunoglobulin, as well as a pharmaceutical composition for the treatment or prevention of a disease caused by the binding between C1q and an immunoglobulin, which includes a peptide capable of binding to the immunoglobulin or a fusion protein with the peptide, and others.

## Description

### Technical Field

The present invention relates to a peptide capable of binding to an immunoglobulin, a fusion protein with the peptide, nucleic acids coding for the peptide and for the fusion protein, methods for producing the peptide and the fusion protein, and a composition and means for binding an immunoglobulin. The present invention also relates to a pharmaceutical composition for the treatment or prevention of a disease caused by the binding between C1q and an immunoglobulin, which includes a peptide capable of binding to the immunoglobulin or a fusion protein with the peptide, and others.

### Background Art

C1q is a complement protein, which is known to act in the pathway of complement activation. For example, the activation of the classical pathway is caused by binding of C1q to the Fc region of immunoglobulin molecules.

It has been reported that patients with rheumatoid arthritis (RA) having a large amount of C1q present in the blood will lead to joint destruction in the future (non-patent document 1) . The above-described activation by C1q is suggested to be involved in joint destruction, and thus there is a need for developing an inhibitor of the binding between C1q and an immunoglobulin molecule.

There is a report that an arginine residue on the C1q B-subunit (B-chain) might be involved in the binding between C1q and immunoglobulin molecules (non-patent document 2). However, this report was based on a prediction by computer simulation, and the actual immunoglobulin-binding site was not identified. In addition, no detailed amino acid sequence of the binding site was revealed.

Proteins, such as Protein A or Protein G, which bind specifically to immunoglobulins, are used in purification of immunoglobulins. However, because these proteins strongly bind to immunoglobulins, severe conditions, such as using strongly acidic buffers are required for the separation of an immunoglobulin after the immunoglobulin is bound to the protein. For this reason, the conformational structure of the immunoglobulin tends to be unfolded, and a high-affinity antibody has not been able to be purified.

### Prior Art Documents

### Non-patent documents

Non-patent document 1: Ochi T et al., Arthritis Rheum., 1988 Jan; 31(1): 37-73
Non-patent document 2: Gaboriaud C et al., J Biol Chem., 2003 Nov 21; 278(47): 46974-82. Epub 2003 Sep 5

### Summary of the Invention

### Problems to be Solved by the Invention

An object to be achieved by the present invention is to provide a peptide capable of binding to an immunoglobulin, a fusion protein with the peptide, nucleic acids coding for the peptide and for the fusion protein, vectors including such nucleic acids, and others. Also provided by the present invention is a novel method for antibody purification, and a composition and means for such a method, by which an antibody having high affinity for an antigen can also be purified without disrupting the conformational structure of the antibody.

### Means for Solving the Problems

In view of the above-described situation, the inventors have made much study and research, and reached the successful identification of the amino acid sequence involved in the binding between C1q and an immunoglobulin, thereby completed the present invention. Surprisingly, this amino acid sequence does not include the arginine residues which have been reported to participate in binding to immunoglobulins. Furthermore, peptides including this amino acid sequence bind to an immunoglobulin weaker than Protein A and Protein G bind, and thus these peptides eliminate problems associated with conventional purification of antibodies, such as unfolding of the conformational structure of antibodies and limiting of antibodies to be purified (for example, antibodies having high affinity for an antigen cannot be purified), so that purification of antibodies has become possible under mild conditions.

Therefore, the present invention is directed to:
(1) a peptide capable of binding to an immunoglobulin, wherein the peptide is selected from the group consisting of:
   (a) a peptide having the amino acid sequence depicted in any of SEQ ID NOs: 1 to 7;
   (b) a peptide having an amino acid sequence in which one or more amino acids are deleted, substituted, or added in the amino acid sequence depicted in any of SEQ ID NOs: 1 to 7; and
   (c) a peptide having an amino acid sequence of 66.7% or more homology to the amino acid sequence depicted in any of SEQ ID NOs: 1 to 7,
      and wherein cysteine(s) in the peptide may be optionally substituted by different kind(s) of amino acid(s);
(2) the peptide according to (1), wherein cysteine(s) in the peptide is(are) substituted by serine(s);
(3) the peptide according to (2), which has the amino acid sequence depicted in any of SEQ ID NOs: 28 to 32;
(4) a peptide capable of binding to an immunoglobulin,
   wherein the peptide is selected from the group consisting of:
   (a) a peptide having the amino acid sequence depicted in any of SEQ ID NOs: 1 to 7;
   (b) a peptide having an amino acid sequence in which one or more amino acids are deleted, substituted, or added in the amino acid sequence depicted in any of SEQ ID NOs: 1 to 7; and
   (c) a peptide having an amino acid sequence of 66.7% or more homology to the amino acid sequence depicted in any of SEQ ID NOs: 1 to 7,
      and wherein amino acid(s) in the peptide may be optionally substituted by the corresponding D-amino acid(s);
(5) the peptide according to (4), wherein all amino acids are substituted by the corresponding D-amino acids;
(6) the peptide according to (5), which has the amino acid sequence represented by dPro-Gly-dLeu-dTyr-dTyr-dPhe;
(7) a peptide capable of binding to an immunoglobulin, wherein the peptide is selected from the group consisting of:
   (a) a peptide having the amino acid sequence depicted in any of SEQ ID NOs: 1 to 7;
   (b) a peptide having an amino acid sequence in which one or more amino acids are deleted, substituted, or added in the amino acid sequence depicted in any of SEQ ID NOs: 1 to 7; and
   (c) a peptide having an amino acid sequence of 66.7% or more homology to the amino acid sequence depicted in any of SEQ ID NOs: 1 to 7,
   and wherein cysteine(s) in the peptide may be optionally substituted by different kind(s) of amino acid(s), and amino acid(s) in the peptide may be optionally substituted by the corresponding D-amino acid(s);
(8) the peptide according to (7), wherein cysteine(s) in the peptide is substituted by different kind(s) of amino acid(s), and all amino acids are substituted by the corresponding D-amino acids;
(9) a nucleic acid coding for a peptide capable of binding to an immunoglobulin, wherein the nucleic acid is selected from the group consisting of:
   (a) a nucleic acid coding for the peptide according to (1);
   (b) a nucleic acid having the nucleotide sequence depicted in any of SEQ ID NOs: 33 to 37;
   (c) a nucleic acid having a nucleotide sequence in which one or more nucleotides are deleted, substituted, or added in the nucleotide sequence depicted in any of SEQ ID NOs: 33 to 37;
   (d) a nucleic acid hybridizable to the nucleic acid of (b) or (c) as described above or the complementary strand thereof under stringent conditions; and
   (e) a nucleic acid having a nucleotide sequence of 50% or more homology to the nucleotide sequence depicted in any of SEQ ID NOs: 33 to 37;
(10) the nucleic acid according to (9), which has the nucleotide sequence depicted in any of SEQ ID NOs: 33 to 37;
(11) a vector including the nucleic acid according to (9) or (10);
(12) a fusion protein, which has the peptide according to any of (1) to (3) added at the N-terminus and/or C-terminus of a desired protein;
(13) a fusion protein, which has the peptide according to any of (4) to (8) added at the N-terminus and/or C-terminus of a desired protein;
(14) a nucleic acid coding for the fusion protein according to (12);
(15) a vector including the nucleic acid according to (14);
(16) a cell including the nucleic acid according to (9) or (10), the nucleic acid according to (14), or the vector according to (11) or (15);
(17) a method for producing a peptide capable of binding to an immunoglobulin, wherein the method includes the steps of:
   (a) transforming a cell with the vector according to (11), and
   (b) culturing the cell to produce the peptide;
(18) a peptide capable of binding to an immunoglobulin, which is obtainable by the method according to (17);
(19) a method for producing a fusion protein in which a peptide capable of binding to an immunoglobulin is added at the N-terminus and/or C-terminus of a desired protein, wherein the method comprises the steps of:
   (a) transforming a cell with the vector according to (15), and
   (b) culturing the cell to produce the fusion protein;
(20) the method according to (19), further comprising the step of obtaining the desired protein from the fusion protein;
(21) a fusion protein which is obtainable by the method according to (19) or (20);
(22) a composition for binding an immunoglobulin, which comprises the peptide according to any of (1) to (8) or the fusion protein according to (12) or (13);
(23) the composition according to (22), which is used for determining the presence or the amount of the immunoglobulin, or for isolating the immunoglobulin;
(24) a means for binding an immunoglobulin, wherein the peptide according to any of (1) to (8) or the fusion protein according to (12) or (13) is immobilized;
(25) the means according to (24), which is used for determining the presence or the amount of the immunoglobulin, or for isolating the immunoglobulin;
(26) a method for binding an immunoglobulin, comprising:
   (a) adding to a sample the peptide according to any of (1) to (8) or the fusion protein according to (12) or (13), and
   (b) determining a complex of the peptide or fusion protein and the immunoglobulin;
(27) a kit comprising a peptide capable of binding to an immunoglobulin or a fusion protein including the peptide, for use in the method according to (26);
(28) a pharmaceutical composition for the treatment or prevention of a disease caused by the binding between C1q and an immunoglobulin, wherein the pharmaceutical composition comprises the peptide according to any of (1) to (8) or the fusion protein according to (12) or (13);
(29) the pharmaceutical composition according to (28), wherein the disease is rheumatoid arthritis;
(30) the pharmaceutical composition according to (28), wherein the disease is an immune-complex disease, such as systemic lupus erythematosus (SLE), glomerulonephritis, vasculitis, or arthritis;
(31) the peptide according to any of (1) to (8) or the fusion protein according to (12) or (13), which is labeled; and
(32) a method for detecting an antibody in a sample, which includes reacting the labeled peptide or fusion protein according to (31) with an antibody in a sample, and then detecting the peptide or fusion protein bound to the antibody.

### Effects of the Invention

The present invention provides a peptide capable of binding to an immunoglobulin, a fusion protein with the peptide, nucleic acids coding for the peptide and for the fusion protein, methods for producing the peptide and the fusion protein, a composition and means for binding an immunoglobulin, and a pharmaceutical composition for the treatment or prevention of a disease caused by the binding between C1q and an immunoglobulin, the pharmaceutical composition including the peptide capable of binding to the immunoglobulin or the fusion protein with the peptide, and others.

### Brief Description of the Drawings

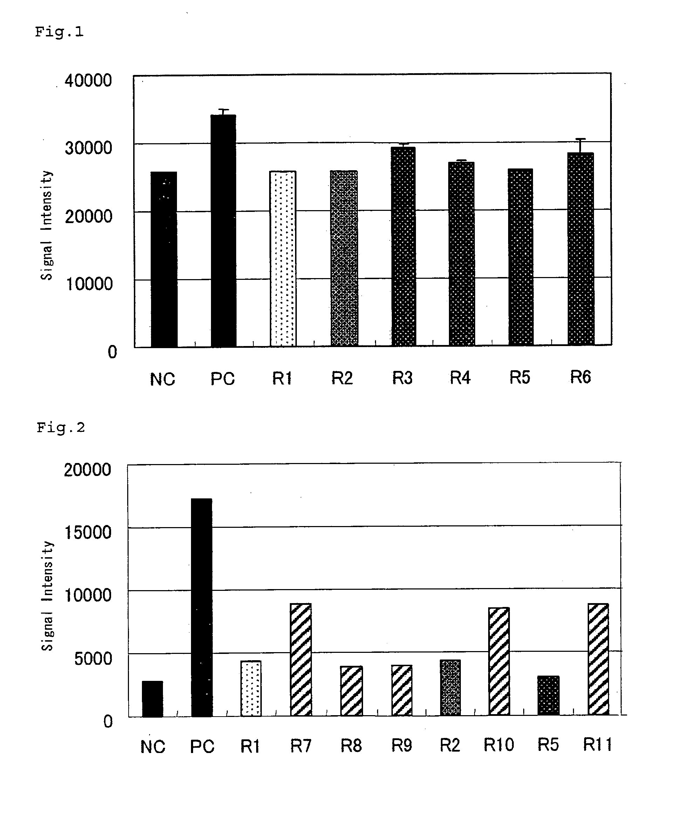

Fig. 1 shows that peptide R1 having six amino acids, peptide R2 having nine amino acids, and peptides R3 to R6 having 15 amino acids inhibit the binding between C1q and an immunoglobulin. In the figure, NC represents the result of samples in which DMSO containing no peptide was added to reaction solutions not containing human immunoglobulin (IgG) labeled with alkaline phosphatase (ALP), and PC in which DMSO containing no peptide was added to reaction solutions containing human immunoglobulin (IgG) labeled with alkaline phosphatase (ALP).
Fig. 2 shows that mutant peptides R7 to R9 having six amino acids, mutant peptide R10 having nine amino acids, and mutant peptide R11 having 15 amino acids inhibit the binding between C1q and an immunoglobulin. In the figure, NC represents the result of samples in which DMSO containing no peptide was added to reaction solutions not containing human immunoglobulin (IgG) labeled with alkaline phosphatase (ALP), and PC in which DMSO containing no peptide was added to reaction solutions containing human immunoglobulin (IgG) labeled with alkaline phosphatase (ALP).

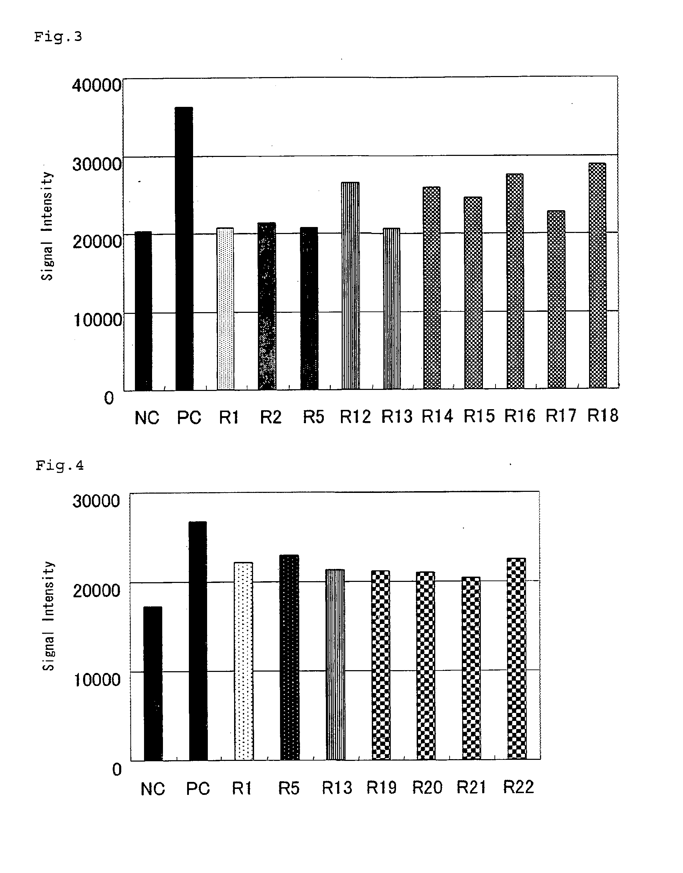

Fig. 3 shows that mutant peptides R12 to R18 inhibit the binding between C1q and an immunoglobulin. In the figure, NC represents the result of samples in which DMSO containing no peptide was added to reaction solutions not containing human immunoglobulin (IgG) labeled with alkaline phosphatase (ALP), and PC in which DMSO containing no peptide was added to reaction solutions containing human immunoglobulin (IgG) labeled with alkaline phosphatase (ALP).
Fig. 4 shows that mutant peptides R19 to R22 inhibit the binding between C1q and an immunoglobulin. In the figure, NC represents the result of samples in which DMSO containing no peptide was added to reaction solutions not containing human immunoglobulin (IgG) labeled with alkaline phosphatase (ALP), and PC in which DMSO containing no peptide was added to reaction solutions containing human immunoglobulin (IgG) labeled with alkaline phosphatase (ALP).

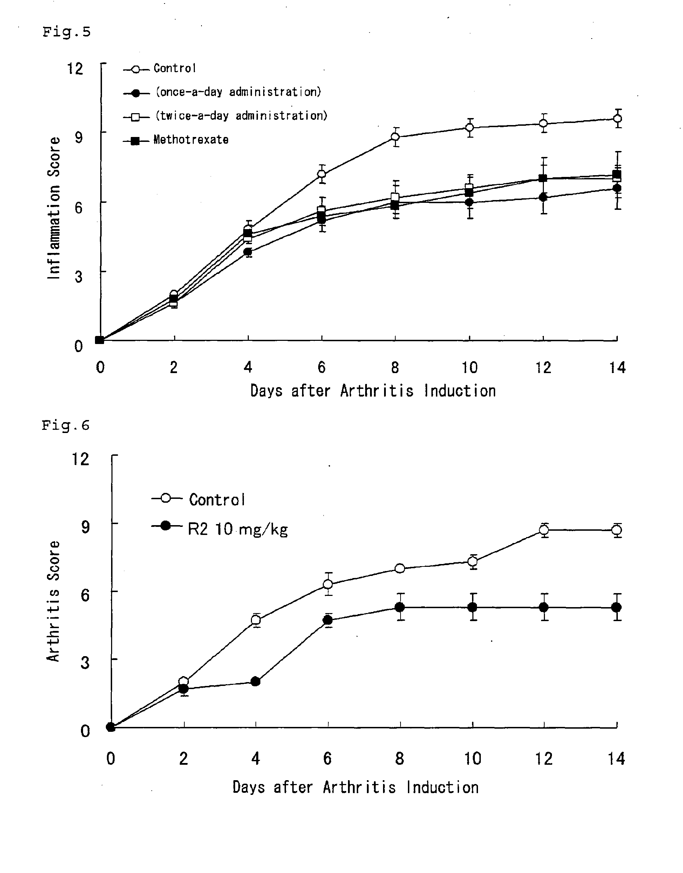

Fig. 5 shows that arthritis was suppressed in arthritis-induced mice by intraperitoneal administration of 10 mg/kg of peptide R5 (once or twice a day). As controls, the results are shown for mice receiving administration of a 0.5% methyl cellulose solution containing no peptide once a day and for mice receiving administration of a methotrexate solution at a dose of 0.1 mg/kg once a day.
Fig. 6 shows that arthritis was suppressed in arthritis-induced mice by intraperitoneal administration of 10 mg/kg of peptide R1. As a control, the result is shown for mice receiving administration of a 0.5% methyl cellulose solution containing no peptide in a similar way.

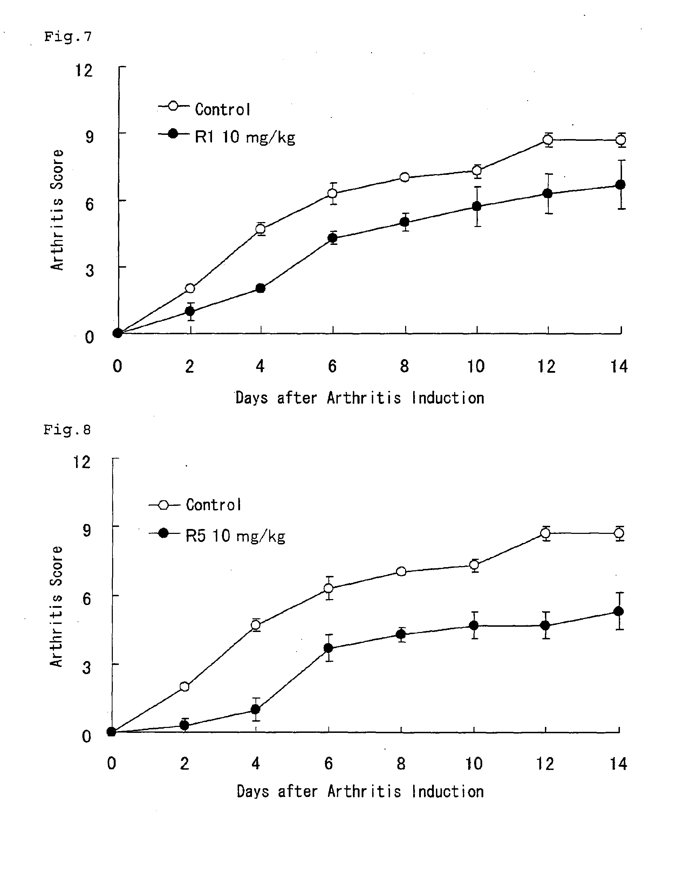

Fig. 7 shows that arthritis was suppressed in arthritis-induced mice by intraperitoneal administration of 10 mg/kg of peptide R2. As a control, the result is shown for mice receiving administration of a 0.5% methyl cellulose solution containing no peptide in a similar way.
Fig. 8 shows that arthritis was suppressed in arthritis-induced mice by intraperitoneal administration of 10 mg/kg of peptide R5. As a control, the result is shown for mice receiving administration of a 0.5% methyl cellulose solution containing no peptide in a similar way.

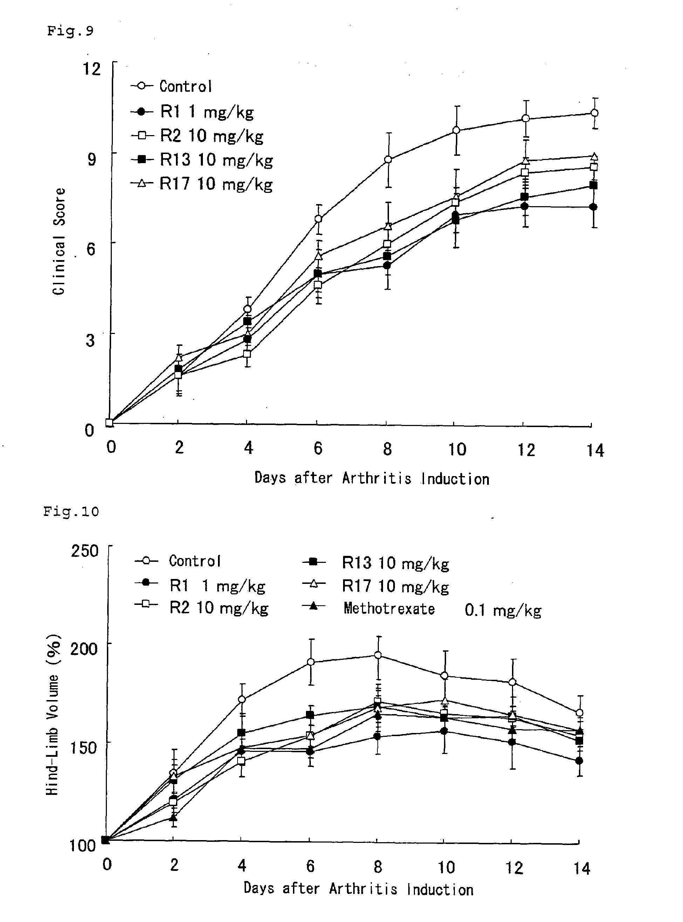

Fig. 9 shows the changes of clinical score in arthritis-induced mice receiving intraperitoneal administration of 10 mg/kg of peptide R2 or R13 or intravenous administration of 1 mg/kg of R1 or 10 mg/kg of R17. As a control, the result is shown for mice receiving administration of physiological saline containing no peptide in a similar way.
Fig. 10 shows the changes of hind-limb volume in arthritis-induced mice receiving intraperitoneal administration of 10 mg/kg of peptide R2 or R13 or intravenous administration of 1 mg/kg of R1 or 10 mg/kg of R17. As controls, the results are shown for mice receiving intraperitoneal administration of physiological saline containing no peptide or of 0.1 mg/kg of methotrexate.

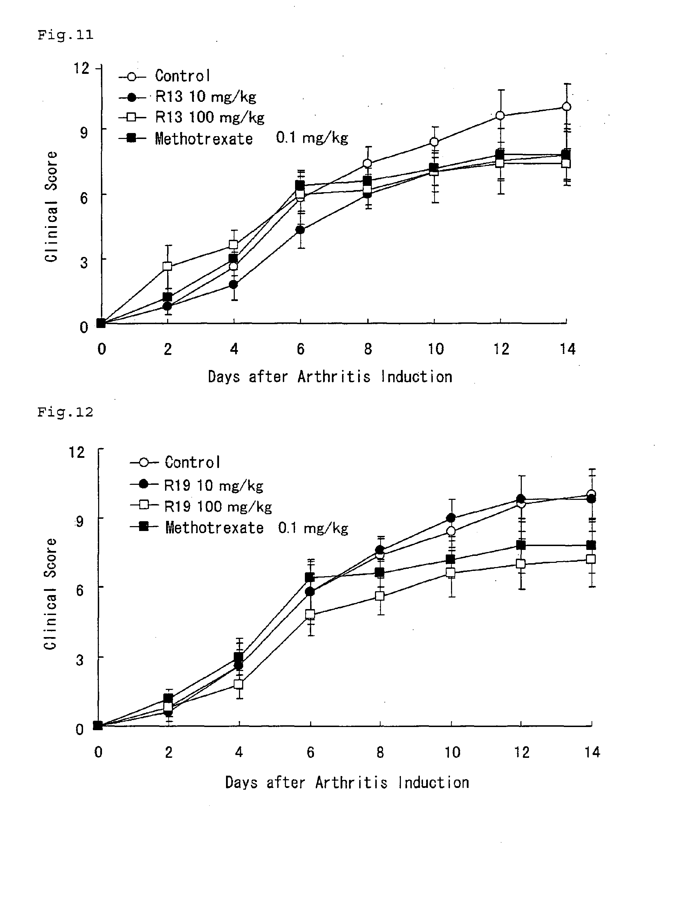

Fig. 11 shows the changes of clinical score in arthritis-induced mice receiving intraperitoneal administration of 10 or 100 mg/kg of peptide 13. As controls, the results are shown for mice receiving intraperitoneal administration of physiological saline containing no peptide or of 0.1 mg/kg of methotrexate.
Fig. 12 shows the changes of clinical score in arthritis-induced mice receiving intraperitoneal administration of 10 or 100 mg/kg of peptide 19. As controls, the results are shown for mice receiving intraperitoneal administration of physiological saline containing no peptide or of 0.1 mg/kg of methotrexate.

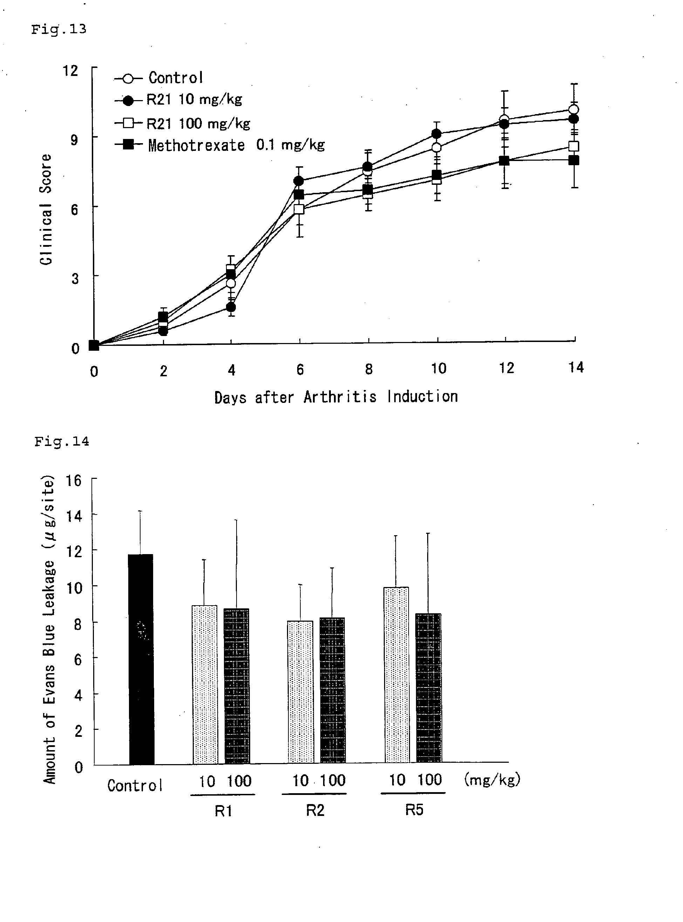

Fig. 13 shows the changes of clinical score in arthritis-induced mice receiving intraperitoneal administration of 10 or 100 mg/kg of peptide 21. As controls, the results are shown for mice receiving intraperitoneal administration of physiological saline containing no peptide or of 0.1 mg/kg of methotrexate.
Fig. 14 shows that an immune-complex disease, such as SLE, glomerulonephritis, vasculitis, and arthritis, was suppressed in type III allergic (Arthus) reaction-induced rats by intraperitoneal administration of 10 or 100 mg/kg of peptide R1, R2, or R5. As a control, the result is shown for rats receiving administration of physiological saline containing no peptide in a similar way.

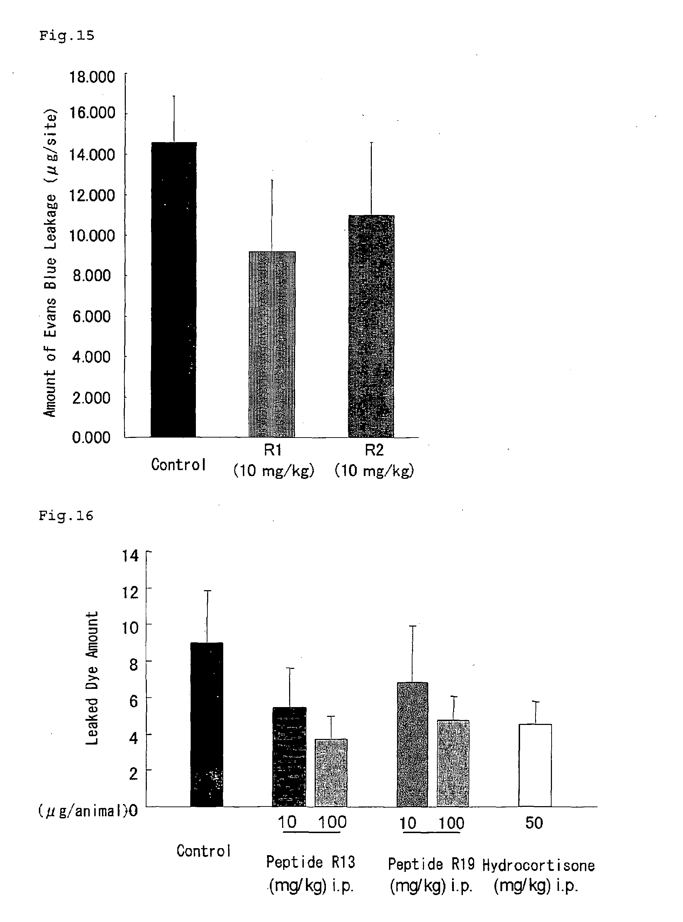

Fig. 15 shows that an immune-complex disease, such as SLE, glomerulonephritis, vasculitis, and arthritis, was suppressed in type III allergic (Arthus) reaction-induced rats by administration of 10 mg/kg of peptide R1 or R2 in the tail vein. As a control, the result is shown for rats receiving administration of physiological saline containing no peptide in a similar way.
Fig. 16 shows that an immune-complex disease, such as SLE, glomerulonephritis, vasculitis, and arthritis, was suppressed in type III allergic (Arthus) reaction-induced rats by intraperitoneal administration of 10 or 100 mg/kg of peptide R13 or R19. As a negative control, the result is shown for rats receiving administration of physiological saline containing no peptide in a similar way. As a positive control, the result is shown for rats receiving administration of 50 mg/kg of hydrocortisone in a similar way.

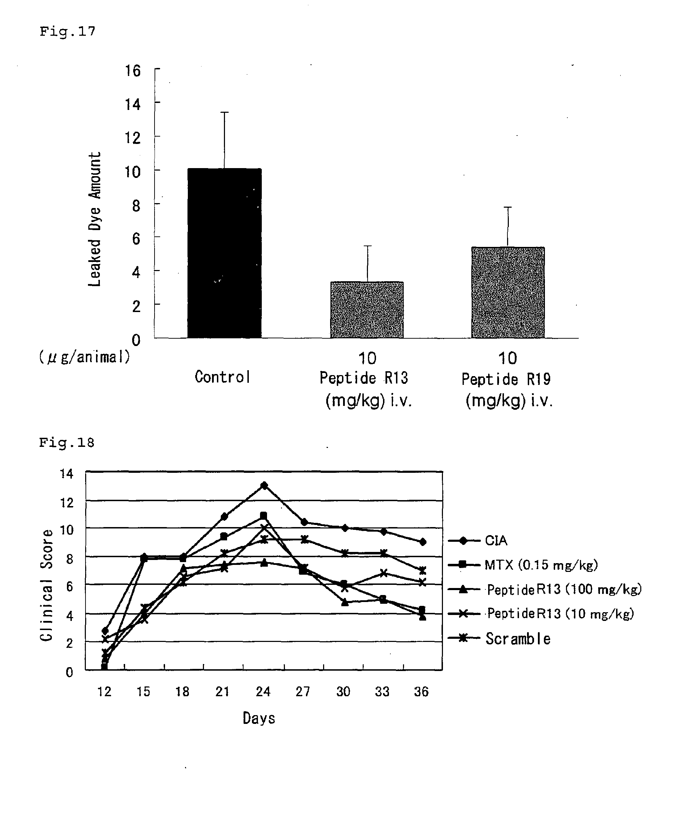

Fig. 17 shows that an immune-complex disease, such as SLE, glomerulonephritis, vasculitis, and arthritis, was suppressed in type III allergic (Arthus) reaction-induced rats by administration of 10 mg/kg of peptide R13 or R19 in the tail vein. As a negative control, the result is shown for rats receiving administration of physiological saline containing no peptide in a similar way.
Fig. 18 shows that arthritis suppressing effects were observed by administration of 100 or 10 mg/kg of the R13 peptide. As negative controls, the results are shown for groups receiving administration of physiological saline or a scramble peptide in a similar way (represented by "CIA" or "scramble" in the figure, respectively). As a positive control, the result is shown for the group receiving administration of methotrexate in a similar way (represented by "MTX (0.15 mg/kg)" in the figure).

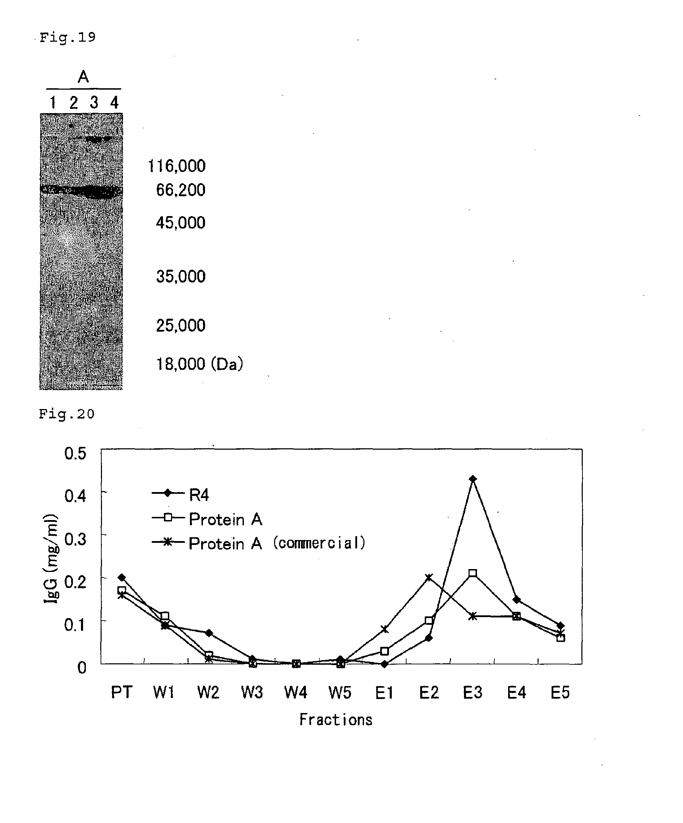

Fig. 19 shows that the peptide of the present invention allowed the detection of an antibody. Each lane used BSA of the following amount: lane 1, 0.1 µg BSA; lane 2, 0.5 µg BSA; lane 3, 1.0 µg BSA; and lane 4, 2.0 µg BSA.
Fig. 20 shows that a rabbit antibody could be purified with an affinity purification column using the peptide of the present invention. As a control, the result of purification using Protein A is shown. The values indicate the amount of IgG protein contained in each fraction (in mg/ml). In the figure, PT represents a flow-through fraction, W1 to W5 represent wash fractions, and E1 to E5 represent elution fractions.

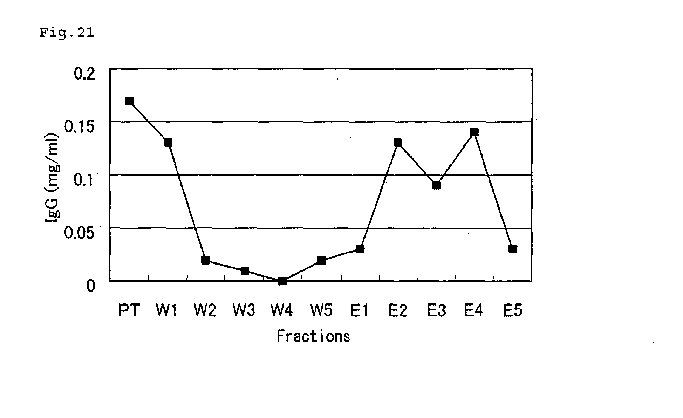

Fig. 21 shows that a human antibody could be purified by an affinity purification column using the peptide of the present invention. The values indicate the amount of IgG protein contained in each fraction (in mg/ml). In the figure, PT represents a flow-through fraction, W1 to W5 represent wash fractions, and E1 to E5 represent elution fractions.

### Preferred Embodiments for Carrying Out the Invention

The present invention, in one aspect, is directed to a peptide capable of binding to an immunoglobulin, wherein the peptide is selected from the group consisting of:
(a) a peptide having the amino acid sequence depicted in any of SEQ ID NOs: 1 to 7;
(b) a peptide having an amino acid sequence in which one or more amino acids are deleted, substituted, or added in the amino acid sequence depicted in any of SEQ ID NOs: 1 to 7; and
(c) a peptide having an amino acid sequence of 66.7% or more homology to the amino acid sequence depicted in any of SEQ ID NOs: 1 to 7,
   and wherein cysteine(s) in the peptide may be optionally substituted by different kind(s) of amino acid(s), and/or amino acid(s) in the peptide may be optionally substituted by the corresponding D-amino acid(s). The peptide of the present invention has a lower binding affinity to an immunoglobulin compared to Protein A and Protein G, which can lead to, for example, dissociation under mild conditions of the immunoglobulin bound to the peptide of the present invention, or the like. In addition, when an immunoglobulin is bound to the peptide of the present invention, denaturation of the immunoglobulin itself is also reduced. Binding affinity can be evaluated by methods known in the art. For example, peptides may be incubated in the presence of an immunoglobulin and determined directly as to whether they bind to the immunoglobulin.

Peptides of the present invention are those in which cysteine(s) may be optionally substituted by different kind(s) of amino acid(s). Amino acids of different classes include not only the L-form and D-form of amino acids, such as glycine, alanine, valine, leucine, isoleucine, serine, threonine, aspartic acid, glutamic acid, asparagine, glutamine, lysine, arginine, cysteine, methionine, phenylalanine, tyrosine, tryptophan, histidine, and proline, but also the L-form and D-form of non-natural amino acids, such as β-alanine, γ-aminobutyric acid (GABA), 5-aminolevulinic acid, 5-aminovaleric acid, homocysteine, ornithine, 5-hydroxytryptophan, 3,4-dihydroxyphenylalanine (dopa), triiodotyronine, thyroxine, homolysine, norleucine, creatine, desmosine, norvaline, and iodotyrosine. Preferably, peptides of the present invention are those in which cysteine(s) has been substituted by serine(s). Peptides of the present invention are those in which amino acid(s) may be optionally substituted by the corresponding D-amino acid(s). Specifically, in the amino acid sequence depicted in any of SEQ ID NOs: 1 to 7, one or more amino acids, preferably, one or some amino acids, for example, 2, 3, 4, 5, 6, 7, 8, 9, 10, 11, 12, 13, 14, or 15 amino acids may be substituted by their corresponding D-amino acids. The corresponding D-amino acids may be the D-form of the above-described non-natural amino acids, in addition to the D-form of amino acids, such as alanine, valine, leucine, isoleucine, serine, threonine, aspartic acid, glutamic acid, asparagine, glutamine, lysine, arginine, cysteine, methionine, phenylalanine, tyrosine, tryptophan, histidine, and proline. Preferably, peptides according to the invention of the present application are those in which all amino acids are substituted by the corresponding D-amino acids. Alternatively, peptides of the present invention are those in which cysteine(s) may be optionally substituted by different kind(s) of amino acid(s) as described above, and amino acid(s) in the peptide may be optionally substituted by the corresponding D-amino acid(s) as described above. Preferably, peptides of the present invention are those in which cysteine(s) has(have) been substituted by serine(s) and all amino acids have been substituted by the corresponding D-amino acids. Peptides of the present invention are ones capable of binding to an immunoglobulin, even though they have any amino acid sequence. Further, peptides of the present invention are resistant to proteolytic degradation and have enhanced stabilities and prolonged half-lives in subjects to whom the peptide has been administered, by modifying native protein-derived amino acid sequences.

Peptides of the present invention are those which are capable of binding to an immunoglobulin. Such peptides may be a peptide which has the amino acid sequence depicted in any of SEQ ID NOs: 28 to 32 or represented by dPro-Gly-dLeu-dTyr-dTyr-dPhe; a peptide which has an amino acid sequence in which one or more amino acids, preferably one or some amino acids, for example, 2, 3, 4, 5, 6, 7, 8, 9, 10, 11, 12, 13, or 14 amino acids are deleted, substituted, or added at the inside, the N-terminus, and/or the C-terminus of the above-described amino acid sequence (mutant peptide); and a peptide which has an amino acid sequence, for example, of 26.6% or more or 44.4% or more, preferably at least 50%, more preferably, for example, 60, 66.7, 70, 75, 80, 83.3, 85, 90, 93% or more homology to the above-described amino acid sequence. In the description, "dL (dLeu)", "dP (dPro)", "dA (dAla)", "dY (dTyr)", and "dF (dPhe)", "dS (dSer)", "dK (dLys)", "dV (dVal)", "dT (dThr)," and "dH (dHis)" stand for the D-form of leucine, proline, alanine, tyrosine, phenylalanine, serine, lysine, valine, threonine, and histidine, respectively. The homology between amino acid sequences can be calculated by simply comparing the sequences in the case of short chain peptides. Alternatively, FASTA, BLAST, DNASIS (Hitachi Software Engineering Co., Ltd.), or GENETYX (Genetyx Corporation) may be used to determine the homology between amino acid sequences. Moreover, any of these amino acids may be modified as appropriate. Peptides of the present invention are ones capable of binding to an immunoglobulin, even though they have any amino acid sequence.

Peptides of the present invention may be of any type, when they have the amino acid sequence as described above. For example, peptides of the present invention may be the very peptide consisting of the amino acid sequence depicted in any of SEQ ID NOs: 28 to 32 or the amino acid sequence represented by dPro-Gly-dLeu-dTyr-dTyr-dPhe. Alternatively, peptides of the present invention also may be ones which include the amino acid sequence as described above or a homologous sequence thereof as a core sequence and has various substances, such as peptides or amino acids, analogs thereof, polyethylene glycols, sugars, polysaccharides, and nucleotides, coupled at the N-terminus and/or C-terminus of said amino acid sequence. Substances, such as fluorescent labels, biotin, streptavidin, digoxigenin (DIG), magnetic beads, latex beads, and gold colloids, may be coupled at the N/C terminus through amino acids or peptides or at the inside of the amino acid sequence through available functional groups. For example, when an additional peptide is coupled, such a peptide may consist of 1 to 50 amino acids, for example, 1 to 20, 1 to 15, 1 to 9 amino acids. In addition, such a peptide may has a function, such as serving as a histidine tag, a GST tag, an S tag, a Myc tag, an HA tag, or an E tag.

Peptides of the present invention can be produced and obtained by various methods known to those skilled in the art. For example, the peptide may be produced by genetic engineering, based on the nucleotide sequence coding for the peptide of the present invention, or chemically synthesized by means of peptide solid-phase synthesis and the like, or produced and obtained in their combination. Peptides of the present invention, which as described above, are capable of binding to an immunoglobulin, can be used to bind an immunoglobulin, thereby determining the presence or the amount of the immunoglobulin, isolating the immunoglobulin, and the like.

The present invention, in another aspect, is directed to a nucleic acid coding for a peptide capable of binding to an immunoglobulin, wherein the nucleic acid is selected from the group consisting of:
(a) a nucleic acid coding for a peptide which has the amino acid sequence depicted in any of SEQ ID NOs: 28 to 32 or a homologous sequence thereof;
(b) a nucleic acid having the nucleotide sequence depicted in any of SEQ ID NOs: 33 to 37;
(c) a nucleic acid having a nucleotide sequence in which one or more nucleotides are deleted, substituted, or added in the nucleotide sequence depicted in any of SEQ ID NOs: 33 to 37;
(d) a nucleic acid hybridizable to the nucleic acid of (b) or (c) as described above or the complementary strand thereof under stringent conditions; and
(e) a nucleic acid having a nucleotide sequence of 50% or more homology to the nucleotide sequence depicted in any of SEQ ID NOs: 33 to 37.
   In the description, a nucleic acid means a single- or double-stranded DNA or RNA. The nucleic acid of the present invention can be produced and obtained by various methods known to those skilled in the art. In the present invention, the nucleotide sequences of SEQ ID NOs: 33 to 37 code for the amino acid sequences of SEQ ID NOs: 28 to 32, respectively.

Specifically, nucleic acids of the present invention are, for example, (1) a nucleic acid coding for a peptide which has the amino acid sequences depicted in any of SEQ ID NOs: 28 to 32; (2) a nucleic acid coding for a peptide which has an amino acid sequence in which one or more amino acids, preferably one or some amino acids, for example, 2, 3, 4, 5, 6, 7, 8, 9, 10, 11, 12, 13, 14 amino acids or so are deleted, substituted, or added in the amino acid sequence depicted in any of SEQ ID NOs: 28 to 32; (3) a nucleic acid coding for a peptide which has an amino acid sequence, for example, of 26.6% or more or 44.4% or more, preferably at least 50%, more preferably, for example, 60, 66.7, 70, 75, 80, 83.3, 85, 90, 93% or more homology to the amino acid sequence depicted in any of SEQ ID NOs: 28 to 32; (4) a nucleic acid having the nucleotide sequence depicted in any of SEQ ID NOs: 33 to 37; (5) a nucleic acid having a nucleotide sequence in which one or more nucleotides, preferably one or some nucleotides, for example, 2, 3, 4, 5, 6, 7, 8, 9 nucleotides or so are deleted, substituted, or added in the nucleotide sequence depicted in any of SEQ ID NOs: 33 to 37; (6) a nucleic acid hybridizable to the nucleic acid described above either in (3) or in (4) or the complementary strand thereof under stringent conditions; and (7) a nucleic acid having a nucleotide sequence of at least 50%, preferably, for example, 60, 70, 75, 80, 90, 93, 95, or 97% or more homology to the nucleotide sequence depicted in any of SEQ ID NOs: 33 to 37. Moreover, any of these nucleotides may be modified as appropriate. Nucleic acids of the present invention can code for a peptide capable of binding to an immunoglobulin, even though they have any nucleotide sequence.

The above-mentioned stringent conditions include, for example, conditions as described in J. Sambrook et al., "Molecular Cloning: A Laboratory Manual, Second Edition," 1989, Cold Spring Harbor Laboratory Press, such as, for example, conditions in which after hybridization is done with a given probe at 68 °C in a solution containing 6X SSC, 5X Denhardt's solution, 0.5% SDS, and 100 µg/ml denatured salmon sperm DNA, washing is carried out by changing washing conditions from at room temperature in 2X SSC, 0.1% SDS to at 68°C in 0.1X SSC, 0.5% SDS; washing at 65 °C is carried out twice for 15 minutes each in a solution containing 2X SSPE (which is described in Frederick M. Ausubel et al., "Current Protocols in Molecular Biology", 1987, John Wiley & Sons, Hoboken NJ.) and 0.1% SDS, subsequently twice for 15 minutes each in a solution containing 0.5X SSPE and 0.1% SDS, and then twice for 15 minutes each in a solution containing 0.1X SSPE and 0.1% SDS; or washing at 65 °C is carried out twice for 15 minutes each in a solution containing 2X SSPE, 0.1% SDS and formamide (5 to 500), subsequently twice for 15 minutes each in a solution containing 0.5X SSPE, 0.1% SDS and formamide (5 to 50%), and then twice for 15 minutes each in a solution containing 0.1X SSPE, 0.1% SDS and formamide (5 to 50%) .

The homology to the above-described nucleotide sequences can be determined, for example, using FASTA, BLAST, DNASIS (Hitachi Software Engineering Co., Ltd.), or GENETYX (Genetyx Corporation).

Nucleic acids of the present invention may be of any type, when they have the nucleotide sequence as described above. For example, nucleic acids of the present invention may be the very nucleic acid consisting of the nucleotide sequence depicted in any of SEQ ID NOs: 33 to 37, as well as a nucleic acids which includes as a core sequence, the nucleotide sequence as described above and have various substances, such as nucleotides, polynucleotides, or analogs thereof, coupled at the 5' end and/or 3' end of said sequence. For example, when a polynucleotide is coupled, such a polynucleotide may consist of 1 to 150 nucleotides, for example, 1 to 60, 1 to 45, 1 to 18 nucleotides.

The present invention, in an additional aspect, is directed to a vector including a nucleic acid as described above. Vectors of the present invention may be of any type, when they include the nucleic acid as described above. The type of vectors, sequences other than the nucleotide sequence of the above-described nucleic acid, and the like, may be selected as appropriate, depending on various factors, such as the host species into and the purpose for which the vector is introduced. Vectors of the present invention can also be used, for example, as a vector for the expression of a fusion protein in which a peptide capable of binding to an immunoglobulin is added at the Nor C-terminus of a desired protein, by inserting a nucleotide sequence coding for the desired protein, in frame at the 5' or 3' end of the nucleotide sequence depicted in any of SEQ ID NOs: 33 to 37. In order to facilitate the isolation of the desired protein from the fusion protein, the vector of the present invention may contain a sequence recognized by a protease, such as HRV 3C, thrombin, Factor Xa or enterokinase, between the above-described nucleotide sequence and the site at which the nucleotide sequence coding for the desired protein is inserted. Vectors of the present invention may be introduced into cells to produce proteins, whereby the above-described peptide of the present invention capable of binding to an immunoglobulin can be obtained.

The present invention, in another aspect, is directed to a fusion protein in which a peptide of the present invention capable of binding to an immunoglobulin is added at the N-terminus and/or C-terminus of a desired protein. Fusion proteins of the present invention can be produced and obtained by various methods known to those skilled in the art. Fusion proteins of the present invention, which include a peptide capable of binding to an immunoglobulin, allow such a peptide to be used as a tag sequence, so as to isolate and/or purify the desired protein, for example. In addition, peptides of the present invention, as described above, have a reduced binding-affinity to an immunoglobulin, and consequently fusion proteins of the present invention have advantages: for example, when immobilized on a purification column, the fusion protein will make it possible to purify the immunoglobulin under mild conditions, relative to cases employing Protein A or Protein G, and to use repeatedly such a column (of which the deterioration is suppressed), and when used as a probe for detecting an immunoglobulin, the fusion protein will facilitate reprobing.

Desired proteins which are to be contained in the fusion protein of the present invention may be of any kind. When fusion proteins of the present invention contain, as a desired protein, for example, an enzyme such as alkaline phosphatase (ALP), peroxidase (HRP), luciferase, fluorescent proteins, for example, green fluorescence protein (GFP), or the like, ß-galactosidase, glutathione S-transferase, or maltose binding protein, such fusion proteins will facilitate, for example, the detection of binding between the peptide capable of binding to an immunoglobulin which is contained in the fusion protein and the immunoglobulin. Fusion proteins of the present invention may also contain a tag sequence, such as a histidine tag, a GST tag, an S tag, a Myc tag, an HA tag, or an E tag, a nuclear localization signal, a silica binding protein, Protein A, or the like.

Fusion proteins of the present invention may include a peptide recognized by a protease, between the peptide of the present invention and the desired protein. By including such a peptide, the desired protein can be isolated from the fusion protein without difficulty.

Therefore, the present invention, in an additional aspect, is directed to a nucleic acid coding for the above-described fusion protein.

The present invention is further directed to a vector including a nucleic acid coding for the above-described fusion protein. Vectors of the present invention may be introduced into cells to produce proteins, whereby the fusion protein of the present invention can be obtained.

The present invention, in one aspect, is directed to a cell including a vector including a nucleic acid coding for the peptide as described above capable of binding to an immunoglobulin, a nucleic acid coding for the fusion protein as described above, or these nucleic acids. Cells of the present invention can be generated by transforming host cells, such as, *Escherichia coli,* yeasty insect or animal cells, with the nucleic acid or vector as described above. Peptides or fusion proteins of the present invention can also be produced by culturing cells of the present invention and collecting the produced peptide or peptide-containing fusion protein, the peptide being capable of binding to an immunoglobulin.

The present invention, in an additional aspect, is directed to a method for producing a peptide capable of binding to an immunoglobulin, the method including the steps of:
(a) transforming a cell with a vector including a nucleic acid coding for a peptide capable of binding to an immunoglobulin; and
(b) culturing the cell to produce the peptide. The transformation of cells can be carried out by means and/or methods known to those skilled in the art.

Therefore, the present invention is directed to a peptide capable of binding to an immunoglobulin, which is obtainable by the above-described method for peptide production.

The present invention, in another aspect, is directed to a method for producing a fusion protein in which a peptide capable of binding to an immunoglobulin is added at the N-terminus and/or C-terminus of a desired protein, the method including the steps of:
(a) transforming a cell with a vector including a nucleic acid coding for a fusion protein in which a peptide capable of binding to an immunoglobulin is added at the N-terminus or C-terminus of a desired protein; and
(b) culturing the cell to produce the fusion protein. The method for fusion-protein production of the present invention may further include the step of removing the desired protein from the fusion protein.

Therefore, the present invention is directed to a fusion protein which is obtainable by the above-described method for fusion-protein production, wherein the fusion protein is a fusion protein in which a peptide capable of binding to an immunoglobulin is added at the N-terminus and/or C-terminus of a desired protein.

The present invention, in one aspect, is directed to a composition for binding an immunoglobulin, which includes a peptide capable of binding to an immunoglobulin, or a fusion protein containing such a peptide. Components other than the above-described peptide or fusion protein may be selected as appropriate, depending on various factors, such as the purpose for which the composition is used. Compositions of the present invention, which as mentioned above, include a peptide capable of binding to an immunoglobulin, may be, for example, a composition for determining the presence or the amount of the immunoglobulin, or a composition for isolating the immunoglobulin. Compositions of the present invention will make it possible to determine the amount of an immunoglobulin in samples, to isolate an immunoglobulin from samples, and others.

The present invention, in another aspect, is directed to a means for binding an immunoglobulin, wherein a peptide capable of binding to an immunoglobulin or a fusion protein containing such a peptide is immobilized. Means of the present invention are ones in which the above-described peptide or fusion protein is immobilized on carriers, such as plates, resins, columns, beads, resins containing sugars such as agarose or sepharose, silica substrates, glass (slide glass and others), metals (gold and others), or apatite. Immobilization may be effected with means/methods known to those skilled in the art, such as methods via an amino or carboxyl group of the peptide or protein, via an SH group of an amino acid side chain, by ionic binding, and by hydrophobic binding.

Means of the present invention, which as mentioned above, are those in which a peptide capable of binding to an immunoglobulin is immobilized, includes a means for determining the presence or the amount of the immunoglobulin and a means for isolating the immunoglobulin. Means of the present invention can also be used, for example, for ELISA plates, columns for purification of immunoglobulins, glass arrays for detection, microfluidic systems, SPR sensor chips, silica substrates for detection, systems for purification of pharmaceutical antibodies, and others.

The present invention, in an additional aspect, is directed to a method for binding an immunoglobulin, the method including:
(a) adding to a sample a peptide capable of binding to an immunoglobulin, or a fusion protein containing such a peptide; and
(b) determining a complex of the peptide or fusion protein and the immunoglobulin.
   Samples may be of any kind, when they are likely to contain an immunoglobulin. By determining the existence and/or the amount of a complex with an immunoglobulin, it is possible to determine whether the immunoglobulin is present in samples, and furthermore, the amount of immunoglobulin present in the samples, for example. The peptides or fusion proteins for use in the method of the present invention may be labeled. Labels include various ones known to those skilled in the art, such as biotinylation, fluorescent, RI, or enzyme labels. Attachment of such a label facilitates examining a complex with the peptide or fusion protein. In addition, the method of the present invention may include the step of isolating the immunoglobulin from the complex.

Therefore, the present invention is directed to a kit for use in the method for binding an immunoglobulin, wherein the kit contains a peptide capable of binding to an immunoglobulin or a fusion protein comprising such a peptide. Kits of the present invention may include, for example, a label, a means to determine a complex, and others, in addition to the above-described peptide or fusion protein.

The present invention, in other aspect, is directed to a pharmaceutical composition for the treatment or prevention of a disease caused by the binding between C1q and an immunoglobulin, wherein the pharmaceutical composition include a peptide capable of binding to the immunoglobulin or a fusion protein including such a peptide. The disease caused by the binding between C1q and an immunoglobulin refers to a disease resulted directly or indirectly from said binding and includes, for example, immune-complex diseases, such as rheumatoid arthritis, arthritis, systemic lupus erythematosus (SLE), vasculitic syndrome, and nephritis, other inflammatory diseases, infectious diseases, and malignant tumors. Pharmaceutical composition of the present invention can treat and prevent the above-described diseases through the inhibition of the binding between C1q and an immunoglobulin by the binding of the peptide contained in the composition to the immunoglobulin. Because the peptide of the present invention is derived from C1q which is originally present within the human body, and is short in length with 6 to 15 residues, adverse effects are diminished which arise from the administration of the peptide of the present invention to humans. Furthermore, the peptide of the present invention is resistant to proteolytic degradation and has an enhanced stability and a prolonged half-life in subjects to whom the peptide has been administered, by modifying native protein-derived amino acid sequences.

Methods of administrating pharmaceutical compositions of the present invention may be selected as appropriate, depending on factors, such as the type of diseases, the condition of subjects, and/or the site to be targeted. Methods of administration include, but are limited to, intradermal, subcutaneous, intramuscular, intravenous, transnasal, oral administration, and others. The amount of the peptide contained in pharmaceutical compositions of the present invention, dosage form of the pharmaceutical compositions, frequency of administration, and the like may be selected as appropriate, depending on factors, such as the type of diseases, the condition of subjects, and/or the site to be targeted. For example, the dose of the peptide per administration may be, without limitation to, 0.01 to 100 mg/kg, preferably 0.1 to 10 mg/kg.

The present invention, in an additional aspect, is directed to a method for the treatment or prevention of a disease caused by the binding between C1q and an immunoglobulin, including administering to a subject an effective amount of a peptide capable of binding to an immunoglobulin or a fusion protein containing such a peptide.

The present invention will be described specifically and in detail by way of Examples, which are not to be interpreted as limiting of the present invention.

### Example 1

### Identification of Amino Acid Sequence in C1q Recognized by Immunoglobulin

### Materials and Methods

The amino acid sequences of the A, B, and C subunit chains of human C1q, are shown in SEQ ID NOs: 17 to 19, and the nucleotide sequences in SEQ ID NOs: 20 to 22. Based on each of the amino acid sequences, sequences having 15 amino acids (residues) from each subunit and shifted at intervals of three amino acids, were synthesized on a glass array as synthetic peptides (Peptide Nos. 1 to 78, 97 to 117, and 193 to 270 for the A, B, and C chains, respectively). The synthesis of each peptide was carried out at a specific location on the array, so as to prepare a peptide array which included the synthetic peptides covering the entire amino acid sequences of the subunits of C1q. The preparation of the array was outsourced to JPT Peptide Technologies GmbH.

To the peptide array was applied 330 µ1 of an Cy3-labeled goat anti-mouse immunoglobulin (IgG) (1 mg/ml; Zymed Laboratories) diluted 1000-fold with PBS (10 mM phosphate buffer, pH 7.0, 0.1 M NaCl), and the array was sealed and then incubated at 4 °C for 12 hours. After that, the array was washed once with methanol and then 5 times with Milli-Q water for 5 minutes each. The array slide was centrifuged and dried, and scanned with a fluorescence scanner (Agilent DNA microarray scanner; Agilent Technologies), so that fluorescence intensity was quantified at the respective peptide spots on the array using a software (Feature Extraction software; Agilent Technologies). Several spots displaying strong fluorescence intensities were detected. Table 1 shows the amino acid sequences which correspond to peptide spots, among these spots, displaying fluorescence intensities that were higher than background level by 60,000 or greater (SEQ ID NOs: 3 to 7). Note that in the description, the amino acids are denoted by the one-letter code known in the art.

**Table 1**

| Peptide No. | Amino acid sequence | SEQ ID NO | intensity* intensity |
|---|---|---|---|
| 149 | SGKFTCKVPGLYYFT | 3 | 65,300 |
| 150 | FTCKVPGLYYFTYHA | 4 | 65,311 |
| 244 | STGKFTCKVPGLYYF | 5 | 65,311 |
| 245 | KFTCKVPGLYYFVYH | 6 | 65,311 |
| 246 | CKVPGLYYFVYHASH | 7 | 65,313 |

| | | | |
|---|---|---|---|
| *Background fluorescence intensity is 2.6 | | | |

### Results

Peptides Nos.149 and 150 have sequences derived from the C1q B-subunit (B chain), and Peptides Nos.244 to 246 have sequences derived from the C1q C-subunit (C chain). From these sequences, it was predicted that the sequence which was essential for the binding between the C1q and the immunoglobulin was a sequence having nine residues, CKVPGLYYF (SEQ ID NO: 2), as a core. It was also observed that the binding to the immunoglobulin was not prevented by peptides having sequences including this core sequence, even though the sequences had several amino acid residues attached at the N-terminus or C-terminus of the core sequence. The nucleotide sequences of peptides Nos.149 and 150, and Nos.244 to 246 are shown in SEQ ID NOs: 12 to 16.

### Example 2

Inhibition of Binding between Immunoglobulin and C1q by Peptides Capable of Binding to Immunoglobulin Examination of Inhibitory Activity (1)

### Materials and Methods

An investigation was made on whether the 9-residue peptide CKVPGLYYF (SEQ ID NO: 2), which was supposed to be essential for the binding between the C1q and the immunoglobulin (IgG), inhibited the binding between the C1q and an immunoglobulin. Peptides were synthesized by GL Biochem (Shanghai), which have the following amino acid sequences: the amino acid sequence depicted in SEQ ID NO: 2 (peptide R2); a shorter amino acid sequence than this peptide, PGLYYF (SEQ ID NO: 1) (peptide R1); and amino acid sequences including the amino acid sequence depicted in SEQ ID NO: 2, SGKFTCKVPGLYYFT (SEQ ID NO: 3) (peptide R3), FTCKVPGLYYFTYHA (SEQ ID NO: 4) (peptide R4), STGKFTCKVPGLYYF (SEQ ID NO: 5) (peptide R5), and CKVPGLYYFVYHASH (SEQ ID NO: 7) (peptide R6). These peptides are shown in Table 2. Each peptide was dissolved in DMSO to make a concentration of 10 mg/ml and stored.

**Table 2**

| Synthetic peptide | Amino acid sequence | SEQ ID NO |
|---|---|---|
| R1 | PGLYYF | 1 |
| R2 | CKVPGLYYF | 2 |
| R3 | SGKFTCKVPGLYYFT | 3 |
| R4 | FTCKVPGLYYFTYHA | 4 |
| R5 | STGKFTCKVPGLYYF | 5 |
| R6 | CKVPGLYYFVYHASH | 7 |

Human C1q protein (Carbiochem) was dissolved in 10 mM HEPES, 300 mM NaCl, 40% glycerol (pH 7.2) to prepare a 200 µg/ml solution of human C1q protein. The solution of human C1q protein was spotted at a volume of 2 µ1 (400 ng) per spot onto a nitrocellulose membrane of a size of 5 mm by 15 mm (Hybond C; Amersham), which was then air-dried at room temperature for about one hour. The nitrocellulose membrane was soaked in TBS, incubated for 5 minutes, and blocked at room temperature for one hour using TBS (20 mM Tris-HC1, pH 7.5, 150 mM NaCl) containing 5% of a blocking agent (Amersham ECL blocking reagent; GE Healthcare). After washing lightly in TBS, the membrane was soaked in 20 µ1 of a solution of alkaline phosphatase (ALP)-labeled human immunoglobulin (IgG) (BECKMAN COULTER) diluted 1000-fold with TBS, which was mixed with each peptide solution to make a peptide concentration of 500 µg/ml, and subjected to reaction at room temperature for one hour. After washing lightly in a TTBS solution (TBS to which Tween 20 was added to the final concentration of 0.05%), the membrane was washed in the same solution three times for 10 minutes each, with shaking.

### Detection Method using ALP-Labeled Immunoglobulin (Igg)

After the nitrocellulose membrane was washed lightly in a color development buffer for ALP (Tris-HC1 containing 100 mM NaCl, 5 mM MgCl₂, pH 9.5), the membrane was soaked in 30 µ1 of the color development buffer for ALP containing a BCIP/NBT solution (Promega), and subjected to color development at room temperature for 10 minutes. When color developments were achieved, the nitrocellulose membrane was soaked in a sufficient volume of distilled water to wash away the color development buffer. After washing, the nitrocellulose membrane was air-dried and the color developments were captured using a Multi Gauge (FUJIFILM).

### Results

The results are shown in Fig. 1. It was found from the fact that the binding was inhibited not only by peptide R2, which had the nine amino acids supposed to be essential for the binding between the C1q and the immunoglobulin (IgG), and peptides including this peptide segment as a core sequence (peptides R3 to R6), but also by the peptide having the shorter amino acid sequence (peptide R1) that, as a core, a sequence having six amino acid residues, PGLYYF (SEQ ID NO: 1), was important for the binding between the immunoglobulin and the C1q. It was also found that inhibition of the binding between C1q and an immunoglobulin would have the potential of treating or preventing diseases caused by said binding.

### Examination of Inhibitory Activity (2)

### Materials and Methods

In addition to the peptides demonstrated in the section Examination of inhibitory activity (1) in Example 2, their mutant peptides were investigated on whether they inhibited the binding between a C1q and an immunoglobulin. The peptides listed in Table 3 were synthesized by GL Biochem (Shanghai). Each peptide was dissolved in DMSO to make a concentration of 10 mg/ml and stored. Experiments were carried out in a similar way to those in the section Examination of inhibitory activity (1) in Example 2, and peptides R1, R2, and R5 were also subjected to experiments as controls.

**Table 3**

| Synthetic peptide | Amino acid sequence | SEQ ID NO |
|---|---|---|
| R7 | PGAYYF | 23 |
| R8 | PGLAYF | 24 |
| R9 | PGLYAF | 25 |
| R10 | CKAPGLYYF | 26 |
| R11 | STAKFTCKVPGLYYF | 27 |

### Results

The results are shown in Fig. 2. The peptides in which a specific amino acid was substituted by alanine sufficiently inhibited the binding between the C1q and the immunoglobulin. Therefore, it was found that for these mutant peptides or peptides including these peptide segments as a core, inhibition of the binding between C1q and an immunoglobulin would have the potential of treating or preventing diseases caused by said binding.

### Examination of Inhibitory Activity (3)

### Materials and Methods

Subsequently to the section (2), Example 1, additional mutant peptides were investigated on whether they inhibited the binding between C1q and an immunoglobulin. The peptides listed in Table 4 were synthesized by GL Biochem (Shanghai). For these peptides, peptide stability was improved by substituting a specific amino acid residue by the corresponding D-amino acid, or by substituting a cysteine residue in the amino acid sequence by a serine residue. That is, these peptides were designed to have prolonged half-lives and maintain their therapeutic or prophylactic effects within the body of subjects for extended periods. Each peptide was dissolved in DMSO to make a concentration of 10 mg/ml and stored. Experiments were carried out in a similar way to those in the section Examination of inhibitory activity (1) in Example 2, and peptides R1, R2, and R5 were used as controls.

**Table 4**

| Synthetic peptide | Amino acid sequence | SEQ ID NO |
|---|---|---|
| R12 | PGdLYYF | Corresponding to 1 |
| R13 | dPGdLdYdYdF | Corresponding to 1 |
| R14 | SGKFTSKVPGLYYFT | 28 |
| R15 | STGKFTSKVPGLYYF | 29 |
| R16 | SKVPGLYYFVYHASH | 30 |
| R17 | KFTSKVPGLYYFVYH | 31 |
| R18 | FTSKVPGLYYFTYHA | 32 |

### Results

The results are shown in Fig. 3. In the figure, NC represents the result of samples in which DMSO containing no peptide was added to reaction solutions not containing human immunoglobulin (IgG) labeled with alkaline phosphatase (ALP), and PC in which DMSO containing no peptide was added to reaction solutions containing human immunoglobulin (IgG) labeled with alkaline phosphatase (ALP). The peptides in which a specific amino acid was substituted by the corresponding D-amino acid (R12 and R13) and the peptides in which a serine residue was substituted by a cysteine residue (R14 to R18) each inhibited the binding between the C1q and the immunoglobulin.

### Examination of Inhibitory Activity (4)

### Materials and Methods

With regard to additional mutant peptides, it was investigated whether they inhibited the binding between a C1q and an immunoglobulin. The peptides listed in Table 5 were synthesized by GL Biochem (Shanghai). In the case of these peptides, peptide stability was improved both by substituting an amino acid residue by the corresponding D-amino acid and by substituting a cysteine residue in the amino acid sequence by a serine residue. That is, these peptides were designed to have prolonged half-lives and maintain their therapeutic or prophylactic effects within the body of subjects for extended periods. Each peptide was dissolved in DMSO to make a concentration of 10 mg/ml and stored. Experiments were carried out in a similar way to those in the section Examination of inhibitory activity (1) in Example 2, and peptides R1, R5, and R13 were used as controls.

**Table 5**

| Synthetic peptide | Amino acid sequence | SEQ ID NO |
|---|---|---|
| R19 | dSdKdVdPGdLdYdYdF | Corresponding to 2 |
| R20 | dSGdKdFdTdSdKdVdPGdLdYdYdFdT | Corresponding to 3 |
| R21 | dSdTGdKdFdTdSdKdVdPGdLdYdYdF | Corresponding to 5 |
| R22 | dFdTdSdKdVdPGdLdYdYdFdTdYdHdA | Corresponding to 4 |

### Results

The results are shown in Fig. 4. In the figure, NC represents the result of samples in which DMSO containing no peptide was added to reaction solutions not containing human immunoglobulin (IgG) labeled with alkaline phosphatase (ALP), and PC in which DMSO containing no peptide was added to reaction solutions containing human immunoglobulin (IgG) labeled with alkaline phosphatase (ALP). The peptides, in which an amino acid was substituted by the corresponding D-amino acid, and a cysteine residue in the amino acid sequence was substituted by a serine residue (R19 to R22), each inhibited the binding between the C1q and the immunoglobulin.

### Example 3

Study of Arthritis Suppressing Effects of Peptide capable of Binding to Immunoglobulin (1) Arthritis Suppressing Effects of Peptide Capable of Binding to Immunoglobulin, on Arthritis-Induced Mice, Part 1

### Materials and Methods

Monoclonal antibody cocktail-induced arthritis mice (BALB/c Cr Slc (SPF)) were used to examine arthritis suppressing effects of a peptide which is indicated as R5 in Example 2. Arthritis was caused by intravenous administration of 2 mg/mouse of an arthritis-inducing monoclonal antibody cocktail and three days later, intraperitoneal administration of 50 µg/mouse of Lipopolysaccharide (LPS). Peptide R5 was intraperitoneally administered at a dose of 10 mg/kg once or twice a day from the day following the LPS administration (day 4) up to day 14. Methotrexate, a positive control drug, was orally administered at a dose of 0.1 mg/kg once a day from day 4. Clinical scores of the extremities were measured on even days from day 0 up to day 14. Under anesthesia, blood was removed with heparin-containing physiological saline, and an ice-cooled, 4% solution of paraformaldehyde was continuously injected to perfusion-fix the whole body. After perfusion-fixation, the knee joints (obtained by cutting at the center of the femur and of the tibia and removing the skin and muscles) and hock joints (from the center of the tibia to the toe tips) of both hind limbs were excised and further fixed by immersion in a 4% solution of paraformaldehyde overnight (4°C). After that, both knee joints and both hock joints were transferred into 50 mM PBS (4°C), and then subjected to soft x-ray radiography of the hock joints in two directions (from the internal side and the upper side).

### Preparation of Peptide Solution

The required amount was calculated for the peptide, based on the animal weight. A peptide solution was prepared to make a concentration of 1 mg/ml in a 0.5% methyl cellulose solution, and the prepared peptide solution was cold stored.

### Preparation of Methotrexate Solution

A solution of methotrexate was prepared by weighing 1 mg of methotrexate into an agate mortar, grinding it with a pestle, and then gradually adding a 0.5% methyl cellulose solution to form a suspension and make a concentration of 0.01 mg/ml. Then, the preparation was cold stored.

### Measurement of body weight, observation of general condition, and grouping

Measurement of body weight of animals was made on days 0, 3, 6, 9, 12, and 14 during the study period, with day 0 being the day of administration of the arthritis-inducing monoclonal antibody cocktail. Observation of general condition was made on a daily basis.

### Generation of Arthritis Model

To twenty 7-week-old mice, the arthritis-inducing monoclonal antibody cocktail was intravenously administered at a dose of 2 mg/mouse on day 0, and LPS was intraperitoneally administered at a dose of 50 µg/mouse on day 3.

### Administration of Peptide Solution

The peptide solution was given via intraperitoneal administration at a dose of 10 mg/kg once a day (morning) or twice a day (morning and afternoon) from day 4. As a control, a 0.5% solution of methyl cellulose was intraperitoneally administered once a day (morning) from day 4. A needle (26G; Terumo) and a syringe (1.0-ml capacity; Terumo) were used for administration. The volume of administration was set to 10 ml/kg. Methotrexate was orally administered at a dose of 0.1 mg/kg once a day (morning) from day 4. A peroral probes (peroral probe for mice; Fuchigami Kikai-Ten) and a syringe (1.0-ml capacity; Terumo) were used for administration. The volume of administration was set to 10 ml/kg.

### OBSERVATION OF CLINICAL SCORE

The clinical score of each limb was observed on even days from day 0 up to day 14 according to the following criteria, with the total score for the extremities being set to a maximum of 12.

### <Clinical Scores>

0: normal joint,
1: slight inflammation and redness,
2: serious erythema and swelling over the whole limb and preventing the use of the limb,
3: deformation of the limb or joint accompanied by ankylosis, joint stiffness, loss of function.

### Method for Statistical Analysis

Results of examinations were expressed by mean value ± standard error and analyzed using EXSAS (Version 7.1.6, Arm Systex Co., Ltd.). Clinical scores were subjected to Wilcoxon's test. Visual observation of swelling of the legs was done.

### Results

The results are shown in Fig. 5. Peptide R5 exhibited arthritis suppressing effects, though there was observed no significant difference in the effectiveness of peptide R5 between once-a-day administration and twice-a-day administration. In addition, this peptide exhibited a higher degree of arthritis suppression than that of methotrexate. From these results, the peptide of the present invention was-found to have an arthritis suppressing effect superior to that of the pharmaceutical which is already in clinical use, and to be useful in the treatment and prevention of arthritis and related diseases.
Methotrexate is known to cause serious adverse effects even when used in very small amounts. The peptide of the present invention, on the other hand, is derived from the natural substance and did not display any detectable adverse effects. Therefore, the use of the present invention has advantages, relative to conventionally used pharmaceuticals.

### (2) Arthritis Suppressing Effects of Peptide Capable of Binding to Immunoglobulin, on Arthritis-Induced Mice, Part 2

### Materials and Methods

Monoclonal antibody cocktail-induced arthritis mice were used to examine arthritis suppressing effects of peptides which are indicated as R1, R2, and R5 in Example 2. Arthritis was caused by intravenous administration of 2 mg/mouse of an arthritis-inducing monoclonal antibody cocktail and three days later, intraperitoneal administration of 50 µg/mouse of LPS. The peptides were administered at a dose of 10 mg/kg twice a day for a period of 14 consecutive days from the day of administration of the arthritis-inducing monoclonal antibody cocktail (day 0), and arthritis suppressing effect was examined in terms of clinical score. Experiments were carried out in a similar way to those in the section (1) in Example 3.

### Preparation of Peptide Solutions

The required amount was calculated for each peptide, based on the animal weight. Each peptide solution was prepared to make a concentration of 1 mg/ml in a 0.5% methyl cellulose solution, and the prepared peptide solution was cold stored.

### Measurement of Body Weight and Observation of General Condition

Measurement of body weight of animals was made on days 0, 3, 6, 9, 12, and 14 during the study period, with day 0 being the day of administration of the arthritis-inducing monoclonal antibody cocktail. Observation of general condition was made on a daily basis.

### Generation of Arthritis Model and Grouping

Mice were weighed on the day prior to start of administration and randomly assigned to groups using a grouping software, so that the average body weight values of the groups were approximately equal. To twenty 7-week-old mice, the arthritis-inducing monoclonal antibody cocktail was intravenously administered at a dose of 2 mg/mouse on day 0, and LPS was intraperitoneally administered at a dose of 50 µg/mouse on day 3.

### Administration of Peptide Solutions

Each peptide solution was given via intraperitoneal administration twice a day, morning and afternoon, from day 0. As a control, a 0.5% solution of methyl cellulose was intraperitoneally administered twice a day from day 0. A needle (26G; Terumo) and a syringe (1.0-ml capacity; Terumo) were used for administration. The volume of administration was set to 10 ml/kg.

### Observation of Clinical Score

The clinical score of each limb of the extremities in all cases was observed on even days from day 0, the day of administration of the arthritis-inducing monoclonal antibody cocktail, up to day 14 according to the following criteria, with the total score for the extremities being set to a maximum of 12.

### <Clinical Scores>

0: normal joint,
1: slight inflammation and redness,
2: serious erythema and swelling over the whole limb and preventing the use of the limb,
3: deformation of the limb or joint accompanied by ankylosis, joint stiffness, loss of function.

### Method for Statistical Analysis

Results of examinations were expressed by mean value ± standard error and analyzed using EXSAS (Version 7.1.6, Arm Systex Co., Ltd.). Clinical scores were subjected to Wilcoxon's test. Visual observation of swelling of the legs was done.

### Results

The results are shown in Figs. 6, 7, and 8. Arthritis suppressing effects were observed in all groups receiving administration of 10 mg/kg of R1, R2, or R5 twice a day. Consequently, the peptides of the present invention were found to have arthritis suppressing effects and to be useful in the treatment and prevention of arthritis and related diseases. In addition, none of the peptides used presented abnormalities in general condition and effects on body weight.
The peptides of the present invention did not display any toxic effects in the examinations using mice. Therefore, the use of peptides of the present invention could be expected to reduce adverse effects associated with treatments or prophylaxes, since the peptides of the present invention are derived from C1q which is originally present within the human body, and are short in length, having 6 to 15 residues.

### (3) Arthritis Suppressing Effects of Peptide Capable of Binding to Immunoglobulin, on Arthritis-Induced Mice, Part 3

### Materials and Methods

Monoclonal antibody cocktail-induced arthritis mice were used to examine arthritis suppressing effects of peptides which are indicated as R13 and R17 in the section (3) in Example 2. Arthritis was caused by intravenous administration of 2 mg/mouse of an arthritis-inducing monoclonal antibody cocktail and three days later, intraperitoneal administration of 50 µg/mouse of LPS. The peptides were given via intraperitoneal or intravenous administration at a dose of 10 mg/kg once a day for a period of 10 consecutive days from four days after the administration of the arthritis-inducing monoclonal antibody cocktail, and arthritis suppressing effect was examined in terms of clinical score and hind-limb volume determination.

### Preparation of Peptide Solutions

The required amount was calculated for each peptide, based on the animal weight. Each peptide solution was prepared using physiological saline (at 0.1 mg/ml for R1, 1 mg/ml for R2, and 1 or 10 mg/ml for R13 and R17), and cold stored.

### Measurement of Body Weight and Observation of General Condition

Measurement of body weight of animals was made on days 0, 3, 6, 9, 12, and 14 during the study period, with day 0 being the day of administration of the arthritis-inducing monoclonal antibody cocktail. Observation of general condition was made on a daily basis.

### Generation of an arthritis model and grouping

Mice were weighed on the day prior to start of administration and randomly assigned to groups using a grouping software, so that the average body weight values of the groups were approximately equal. To fifty-five 7-week-old mice, the arthritis-inducing monoclonal antibody cocktail was intravenously administered at a dose of 2 mg/mouse on day 0, and LPS was intraperitoneally administered at a dose of 50 µg/mouse on day 3.

### Administration of Peptide Solutions

Each peptide solution was given via intraperitoneal or intravenous administration once a day from day 4. Additionally, physiological saline as a negative control, and methotrexate (0.01 mg/kg) as a positive control were intraperitoneally administered once a day from day 4. A needle (26G; Terumo) and a syringe (1.0-ml capacity; Terumo) were used for administration. The volume of administration was set to 10 ml/kg.

### Observation of Clinical Score

The clinical score of each limb of the extremities in all cases was observed on even days from day 0, the day of administration of the arthritis-inducing monoclonal antibody cocktail, up to day 14 according to the following criteria, with the total score for the extremities being set to a maximum of 12.

### <Clinical Scores>

0: normal joint,
1: slight inflammation and redness,
2: serious erythema and swelling over the whole limb and preventing the use of the limb,
3: deformation of the limb or joint accompanied by ankylosis, joint stiffness, loss of function.

### Method for Statistical Analysis

Results of examinations were expressed by mean value ± standard error and analyzed using EXSAS (Version 7.1.6, Arm Systex Co., Ltd.). Clinical scores were subjected to Wilcoxon's test. Visual observation of swelling of the legs was done. Quantitative data, such as body weights and leg volumes, were subjected to Bartlett's test, followed by Dunnett's multiple comparison test when the variance was uniform or Steel's multiple comparison test when the variance was not uniform.

### Results

The results are shown in Figs. 9 and 10. The clinical scores in Fig. 9 show that all groups receiving once-a-day administration of R13 (intraperitoneally) or R17 (intravenously) displayed arthritis-suppressing effects comparable to those of the groups receiving administration of R1 (intravenously) or R2 (intraperitoneally), which were comparable or greater effects when compared with the group receiving administration of methotrexate. From the results of hind-limb volume determinations in Fig. 10, it is understood that the peptides according to the invention of the present application suppressed swelling of the arthritis affected areas to degrees comparable to or higher than those of R1 and R2, and additionally that of methotrexate. Therefore, the peptides of the present invention were found to have arthritis suppressing effects and to be useful in the treatment or prevention of arthritis and related diseases.
Methotrexate is known to cause serious adverse effects even when used in very small amounts. The peptides of the present invention, on the other hand, are derived from the natural substance and did not display any detectable adverse effects. Therefore, the use of the present invention has advantages, relative to conventionally used pharmaceuticals.

### (4) Arthritis Suppressing Effects of Peptide Capable Of Binding to Immunoglobulin, on Arthritis-Induced Mice, Part 4

### Materials and Methods

Monoclonal antibody cocktail-induced arthritis mice were used to examine arthritis suppressing effects of peptides which are indicated as R13 in the section (3) in Example 2 and as R19 and R21 in the section (4) in Example 2. Arthritis was caused by intravenous administration of 2 mg/mouse of an arthritis-inducing monoclonal antibody cocktail and three days later, intraperitoneal administration of 50 µg/mouse of LPS. The peptides were given via intraperitoneal or intravenous administration at a dose of 10 mg/kg once a day for a period of 10 consecutive days from four days after the administration of the arthritis-inducing monoclonal antibody cocktail, and arthritis suppressing effect was examined in terms of clinical score and hind-limb volume determination.

### Preparation of Peptide Solutions

The required amount was calculated for each peptide, based on the animal weight. A peptide solution was prepared using physiological saline (at 10 and 1 mg/kg), and cold stored.

### Measurement of Body Weight and Observation of General Condition

Measurement of body weight of animals was made on days 0, 3, 6, 9, 12, and 14 during the study period, with day 0 being the day of administration of the arthritis-inducing monoclonal antibody cocktail. Observation of general condition was made on a daily basis.

### Generation of Arthritis Model and Grouping

Mice were weighed on the day prior to start of administration and randomly assigned to groups using a grouping software, so that the average body weight values of the groups were approximately the equal. To forty 7-week-old mice, the arthritis-inducing monoclonal antibody cocktail was intravenously administered at a dose of 2 mg/mouse on day 0, and LPS was intraperitoneally administered at a dose of 50 µg/mouse on day 3.

### Administration of Peptide Solutions

Each peptide solution was given via intraperitoneal administration once a day from day 4. Additionally, physiological saline as a negative control, and methotrexate (0.1 mg/kg) as a positive control were intraperitoneally administered once a day from day 4. A needle (26G; Terumo) and a syringe (1.0-ml capacity; Terumo) were used for administration. The volume of administration was set to 10 ml/kg or 100 mg/kg.

### Observation of Clinical Score

The clinical score of each limb of the extremities in all cases was observed on even days from day 0, the day of administration of the arthritis-inducing monoclonal antibody cocktail, up to day 14 according to the following criteria, with the total score for the extremities being set to a maximum of 12.

### <Clinical Scores>

0: normal joint,
1: slight inflammation and redness,
2: serious erythema and swelling over the whole limb and preventing the use of the limb,
3: deformation of the limb or joint accompanied by ankylosis, joint stiffness, loss of function.

### Method for Statistical Analysis

Results of examinations were expressed by mean value ± standard error and analyzed using EXSAS (Version 7.1.6, Arm Systex Co., Ltd.). Clinical scores were subjected to Wilcoxon's test. Visual observation of swelling of the legs was done. Quantitative data, such as body weights and leg volumes, were subjected to Bartlett's test, followed by Dunnett's multiple comparison test when the variance was uniform or Steel's multiple comparison test when the variance was not uniform.

### Results

The results are shown in Figs. 11, 12, and 13. When peptide R13 was intraperitoneally administered at a dose of 10 or 100 mg/kg, there was observed an arthritis-suppressing effect comparable to or greater than that of methotrexate. Also, when peptides R19 and R21 were intraperitoneally administered at a dose of 100 mg/kg, there was observed an arthritis-suppressing effect comparable to or greater than that of methotrexate. Therefore, the peptides of the present invention were found to have arthritis suppressing effects and to be useful in the treatment or prevention of arthritis and related diseases.
Methotrexate is known to cause serious adverse effects even when used in very small amounts. The peptides of the present invention, on the other hand, are derived from the natural substance and did not display any detectable adverse effects. Therefore, the use of the present invention has advantages, relative to conventionally used pharmaceuticals.

### Example 4

Study of Effects of Peptide Capable of Binding to Immunoglobulin, on Immune-Complex Disease Suppression (1) Effects of Peptide capable of Binding to Immunoglobulin on Immune-Complex Disease Suppression, in Type III Allergic (Arthus) Reaction-Induced Rats, Part 1

### Materials and Methods

Using sixty-four 7-week-old male Slc:Wistar rats, examinations were made on peptides R1, R2, and R5, in two series of examinations, each using a different method of administration (intraperitoneal administration and tail vein administration). Acclimation was carried out by giving normal solid chow CRF-1 for a period of 9 days or longer. The back of the animals was shaved on the day prior to administration, and a mixed solution of OVA and Evans blue dye was administered into the tail vein on the day of administration. Each peptide solution was administered intraperitoneally or into the tail vein 30 minutes or 50 minutes after the administration of the mixed solution of OVA and Evans blue dye, respectively. For intracutaneous administration of an anti-OVA solution, each peptide solution was intracutaneously administered in the back of animals at a dose of 0.1 ml/site 30 minutes or 10 minutes after the administration of the tested substance in the case of intraperitoneal or intravenous administration, respectively, so as to generate a local Arthus reaction. After the reaction was allowed to be generated for 4 hours, the animals were euthanized, the blood was removed thoroughly, and then the dorsal skin was exfoliated. The Arthus reaction sites on the exfoliated skin were punched out, and the Evans blue dye was extracted from the punched skin overnight. The amount of dye leakage was quantified by measuring absorbance for the Evans blue dye with a spectrophotometer and using a calibration curve (see, H. Okamoto, Y. Iwahisa, and M. Terasawa, "Suppression of the Arthus reaction by Y-24180, a potent and specific antagonist of platelet-activating factor", Agents Actions, 35:149-158 (1992), for experimental procedures used in Example 4).

The peptide solutions which were used in these experiments are as follows:

### <Negative Control Substance>

### Physiological saline

### <Peptide Solutions>

A predetermined amount of each peptide (peptides R1, R2, and R5) was dissolved in physiological saline to make a 20 mg/ml solution. A 2 mg/ml solution was prepared by diluting the 20 mg/ml solution 10-fold with physiological saline. A 5 mg/ml solution was prepared by dissolving each peptide in a predetermined amount in physiological saline to make a prepared solution.

### Arthus Reaction

A mixed solution of OVA and Evans blue dye was administered into the tail vein at a dose of 2 ml/kg. Each peptide solution was administered intraperitoneally or into the tail vein 30 minutes or 50 minutes after the administration of the mixed solution of OVA and Evans blue dye, respectively. For intracutaneous administration of an anti-OVA solution, each peptide solution was intracutaneously administered in the back of animals at a dose of 0.1 mL/site 30 minutes or 10 minutes after the administration of the tested substance in the case of intraperitoneal or intravenous administration, respectively, so as to generate a local Arthus reaction. Intracutaneous administration was conducted at 2 sites for PBS and at 2 sites for the anti-OVA solution per animal. After the reaction was allowed to be generated for 4 hours, the animals were euthanized by decapitation under ether anesthesia, the blood was removed thoroughly, and then the dorsal skin was exfoliated. The Arthus reaction sites on the exfoliated skin were punched out and subjected to dye extraction.

### Dye Extraction and Measurement

Several cuttings were made in the skin strips that were punched out with a punch. The strip was then immersed in 2 ml of a dye extraction solution and stirred with shaking for 10 minutes. Thereafter, the strip was left standing overnight at room temperature. After the strip was allowed to stand overnight, the strip was stirred again with shaking for 10 minutes, followed by centrifugation (1500 rpm, 15 minutes). The supernatant was used as a sample for dye measurement. A UV-1600 (Shimadzu Corporation) was used for dye measurement, which was made at a wavelength of 620 nm. The amount of dye leakage was calculated from a calibration curve of known amounts of the dye.

### Data Analysis and Statistical Processing

The mean value (mean) ± standard deviation (SD) for each group was calculated with body-weight measurements and amounts of dye leakage. For amounts of dye leakage, the value for each animal was determined by subtracting the mean of values obtained for the two sites of administration of PBS from the mean of values obtained for the two sites of administration of the anti-OVA solution. Statistical analysis was performed on amounts of dye leakage, as follows. In the first examination (via intraperitoneal administration of each peptide solution), Bartlett's test of homogeneity of variances was carried out for group 1 versus groups 2 and 3, for group 1 versus groups 4 and 5, and for group 1 versus groups 6 and 7, followed by testing the difference in the mean values between group 1 and each of the other groups by Dunnett's multiple comparison test when there was no difference in the variances, or Steel's multiple comparison test when there was any difference in the variances. In the second examination (via tail vein administration of each peptide solution), Bartlett's test of homogeneity of variances was carried out for group 9 versus groups 10, 11, and 12, followed by testing the difference in the mean values between group 9 and each of the other groups by Dunnett's multiple comparison test when there was no difference in the variances, or Steel's multiple comparison test when there was any difference in the variances. The level of significance was set at 5% in the Bartlett's test of homogeneity of variances and 5% on both sides in the others.

### Results

The results are shown in Figs. 14 and 15 for the intraperitoneal administration and the tail vein administration of each of the peptide solutions, respectively. In the type III allergic (Arthus) reaction model system used in the example, the peptides of the present invention were found to decrease the amount of Evans blue leakage approximately by 30%, compared to the control, and thus would be likely to be effective in suppressing immune complex diseases, such as SLE, glomerulonephritis, arthritis, vasculitis or the like. There were observed clear and satisfactory effects of immune-complex disease suppression for all the peptides R1, R2, and R5 in cases of the intraperitoneal administration of the peptide solutions. Also in cases of the tail vein administration, clear and satisfactory effects of immune-complex disease suppression were observed with the peptides R1 and R2. Therefore, the peptides of the present invention were found to have a clear and satisfactory effect of immune-complex disease suppression and to be useful in the treatment and prevention of immune complex diseases, such as SLE, glomerulonephritis, arthritis, vasculitis or the like.

### (2) Effects of Peptide capable of Binding to Immunoglobulin on Immune-Complex Disease Suppression, in Type III Allergic (Arthus) Reaction-Induced Rats, Part 2

### Materials and Methods

Using forty-five 7-week-old male Slc:Wistar rats, examinations were made on peptides R13 and R19, in two series of examinations, each using a different method of administration (intraperitoneal administration and tail vein administration). Acclimation was carried out by giving normal solid chow CRF-1 for a period of 6 or 8 days. The back of the animals was shaved on the day prior to administration, and a mixed solution of OVA and Evans blue dye was administered into the tail vein on the day of administration. Each peptide solution was administered intraperitoneally or into the tail vein 30 minutes or 50 minutes after the administration of the mixed solution of OVA and Evans blue dye, respectively. For intracutaneous administration of an anti-OVA solution, each peptide solution was intracutaneously administered in the back of animals at a dose of 0.1 ml/site 30 minutes or 10 minutes after the administration of the tested substance in the case of intraperitoneal or intravenous administration, respectively, so as to generate a local Arthus reaction. After the reaction was allowed to be generated for 4 hours, the animals were euthanized, the blood was removed thoroughly, and then the dorsal skin was exfoliated. The Arthus reaction sites on the exfoliated skin were punched out, and the Evans blue dye was extracted from the punched skin overnight. The amount of dye leakage was quantified by measuring absorbance for the Evans blue dye with a spectrophotometer and using a calibration curve.

The peptide solutions which were used in these experiments are as follows:

### <Negative Control Substance>

### Physiological saline

### <Solution for Administration of Positive Control Substance>

A suspension was prepared by placing a predetermined amount of hydrocortisone into a mortar, grinding it with a pestle, and then adding physiological saline to make the final concentration of 10 mg/ml.

### <Peptide Solutions>

A predetermined amount of each peptide (peptides R13 and R19) was dissolved in physiological saline to make a 20 mg/ml solution. A 2 mg/ml solution was prepared by diluting the 20 mg/ml solution 10-fold with physiological saline. A 5 mg/ml solution was prepared by dissolving each peptide in a predetermined amount in physiological saline to make a prepared solution.

### Arthus Reaction

A mixed solution of OVA and Evans blue dye was administered into the tail vein at a dose of 2 ml/kg. Each peptide solution was administered intraperitoneally or into the tail vein 30 minutes or 50 minutes after the administration of the mixed solution of OVA and Evans blue dye, respectively. For intracutaneous administration of an anti-OVA solution, each peptide solution was intracutaneously administered in the back of animals at a dose of 0.1 mL/site 30 minutes or 10 minutes after the administration of the tested substance in the case of intraperitoneal or intravenous administration, respectively, so as to generate a local Arthus reaction. Intracutaneous administration was conducted at 2 sites for PBS and at 2 sites for the anti-OVA solution per animal. After the reaction was allowed to be generated for 4 hours, the animals were euthanized by decapitation under ether anesthesia, the blood was removed thoroughly, and then the dorsal skin was exfoliated. The Arthus reaction sites on the exfoliated skin were punched out and subjected to dye extraction.

### Dye Extraction and Measurement

Several cuttings were made in the skin strips that were punched out with a punch. The skin strip was then immersed in 2 ml of a dye extraction solution and stirred with shaking for 10 minutes. Thereafter, the strip was left standing overnight at room temperature. After the strip was allowed to stand overnight, the strip was stirred again with shaking for 10 minutes, followed by centrifugation (1500 rpm, 15 minutes). The supernatant was used as a sample for dye measurement. A UV-1600 (Shimadzu Corporation) was used for dye measurement, which was made at a wavelength of 620 nm. The amount of dye leakage was calculated from a calibration curve of known amounts of the dye.

### Data processing and statistical processing

The mean value (mean) ± standard deviation (SD) for each group was calculated with body-weight measurements and amounts of dye leakage. For amounts of dye leakage, the value for each animal was determined by subtracting the mean of values obtained for the two sites of administration of PBS from the mean of values obtained for the two sites of administration of the anti-OVA solution. Statistical analysis was performed on amounts of dye leakage, as follows. In the first test (via intraperitoneal administration of tested substances), Bartlett's test of homogeneity of variances was carried out for groups 1, 2, and 3, and for groups 1, 4, and 5, followed by testing the difference in the mean values between group 1 and each of the other groups by Dunnett's multiple comparison test when there was no difference in the variances, or Steel's multiple comparison test when there was any difference in the variances. In the second test (via tail vein administration of tested substances), Bartlett's test of homogeneity of variances was carried out among groups 7, 8, and 9, followed by testing the difference in the mean values between group 7 and each of the other groups by Dunnett's multiple comparison test when there was no difference in the variances, or Steel's multiple comparison test when there was any difference in the variances. The level of significance was set at 5% in the Bartlett's test of homogeneity of variances and 5% on both sides in the others.

### Results

The results are shown in Figs. 16 and 17 for the intraperitoneal administration and the tail vein administration of each of the peptide solutions, respectively. In the examination via intraperitoneal administration, peptide R13 was found to have a significant suppression effect in both the groups receiving 10- and 100-mg/kg administrations, compared to the group receiving administration of the negative control substance. In particular, in the groups receiving 100-mg/kg administration, a remarkably significant suppression effect (suppression of about 60%) was observed, which was a stronger suppression effect than that of the group receiving administration of 50 mg/kg of the positive control substance, hydrocortisone. In the groups receiving administration of peptide R19, the group receiving 10-mg/kg administration displayed decreased values by 25 to 30%, compared to the group receiving administration of the negative control substance, and was found to have suppression effect. The group receiving 100-mg/kg administration was found to have a 50% suppression effect, which was a similar suppression effect to that of the positive control substance. In the second examination via tail vein administration, the group receiving administration of peptide R13 was found to have an about 70% suppression effect, which was a remarkably significant suppression effect, compared to the group receiving administration of the negative control substance. Also, the group receiving administration of peptide R19 was found to have an about 50% suppression effect, compared to the group receiving administration of the negative control substance. These results were a remarkably significant suppression effect, compared to the peptides synthesized in the same sequences and in the L-form. It was borne out that converting of the L-form peptides into the D-form peptides presented a stronger, clear and satisfactory suppression effect on type III allergic (Arthus) reaction, and it was found from these results that the present peptides would be useful in the treatment and prevention of immune complex diseases, such as SLE, glomerulonephritis, arthritis, vasculitis or the like. Hydrocortisone is classified as a steroidal drug and known to cause serious adverse effects, even though it is used in very small amounts. The peptides of the present invention, on the other hand, are derived from the natural substance and did not display any detectable adverse effects. Therefore, the use of the present invention has advantages, relative to conventionally used pharmaceuticals.

### Example 5

### Arthritis Suppressing Effects of Peptide capable of Binding to Immunoglobulin, on Rat Collagen-Arthritis Model

A rat collagen-arthritis model was used to examine arthritis suppressing effects of a peptide, referred to as peptide R13.
Female Lewis rats were primed with 500 µg of type II bovine-derived collagen and boosted with 100 µg of the same collagen at 7 and 14 days post priming, to induce collagen arthritis. Peptide R13, a scramble peptide (dYdPdYGdFdL), or methotrexate was administered once a day for a period of 25 consecutive days from 12 days after arthritis was fully developed, and arthritis suppressing effect was examined in terms of clinical score. Methotrexate was administered at a dose of 0.15 mg/kg as a control drug.

### Procedures for Preparation of Peptide Solutions

The required amount was calculated for each peptide, based on the animal weight. Physiological saline was gradually added to prepare suspensions, to make concentrations of 100 and 10 mg/ml.

### Observation of Clinical Score

The clinical score of each limb of the extremities in all cases was observed every third day from day 12, on which arthritis was developed, up to day 36, with day 0 being the day of priming with type II bovine-derived collagen. Observations were according to the following criteria, with the total score for the extremities being set to a maximum of 12.

### <Clinical Scores>

0: not changed,
1: slight erythema and swelling at the ankle joint or on the dorsum or sole of foot, or erythema and swelling of not more than two toes,
2: erythema and swelling at the ankle joint or on the dorsum or sole of foot, or erythema and swelling of more than two toes,
3: swelling and erythema over the whole limb,
4: maximum swelling and erythema over the whole limb.

### Results

As shown in Fig. 18, arthritis suppressing effects were observed in the groups receiving administration of 100 and 10 mg/kg of the R13 peptide once a day. In particular, the group receiving administration of 100 mg/kg of the R13 peptide exhibited a suppression effect equal to or greater than that of the group receiving administration of methotrexate, while the scramble peptide did not exhibit any suppression effects. Therefore, the peptides of the present invention were found to have arthritis suppressing effects and to be useful in the treatment and prevention of arthritis and related diseases. In addition, abnormalities in general condition and effects on body weight were not observed at either dose of the peptide used. Histological observations also revealed a significant decrease in the expression of TNF due to the administration of the peptide at sites of joint destruction, and it turned out that the induction of osteoclasts was also suppressed. Methotrexate is known to cause serious adverse effects even when used in very small amounts. The peptide of the present invention, on the other hand, is derived from the natural substance and did not display any detectable adverse effects. Therefore, the use of the present invention has advantages, relative to conventionally used pharmaceuticals.

### Example 6

### Test of Antibody Detection Agent using Peptide Capable of binding to Immunoglobulin

### Materials and Methods

A test was made on whether primary antibodies were detectable in western-blot procedures using a biotinylated peptide instead of a secondary antibody. A biotinylated peptide R5 was produced by GL Biochem (Shanghai).

BSA was electrophoresed on 12% SDS-PAGE and transferred onto a PVDF membrane. The PVDF membrane after transfer was blocked in 5% skim milk. The PVDF membrane was immersed in a 2000-fold diluted solution of anti-BSA IgG (rabbit) and subjected to reaction at room temperature for one hour. The membrane was washed three times in TBST. The biotinylated peptide R5 was dissolved in DMSO to make a 10 mg/ml solution, 10 µl of the solution was added to 10 ml of TBST (1000-fold dilution) and subjected to reaction at room temperature for one hour. The membrane was washed three times in TBST. Color development was carried out using an ABC kit (VECTOR laboratories). The solution for color development used nickel sulfate and diaminobenzidine and the substrate used a solution of hydrogen peroxide.

### Results

The results are shown in Fig. 19. The antibody on the PVDF membrane was detectable using the biotinylated peptide of the present invention instead of a secondary antibody. Therefore, the peptide of the present invention that had been biotinylated was found to be useful in the detection of an antibody.

### Example 7

### Test of Antibody Purification Column using Peptide capable of Binding to Immunoglobulin

### Materials and Methods

A test was made on an antibody purification column using a peptide capable of binding to an immunoglobulin. Protein A was used as a control.

### Preparation of Peptide Column

A PD-10 Empty column was filled with 1 ml of NHS-activated Sepharose 4B Fast Flow, which was then washed with 10 ml of 1 mM HCl and equilibrated with 10 ml of PBS. 5 mg of peptide R4 was dissolved in 1 ml of PBS and applied to the column, which was rotated at room temperature for four hours. The column was washed with 5 ml of PBS. After that, 1 ml of 1 M glycine was applied to the column, which was then rotated at room temperature for two hours to block unreacted NHS. The 1 M glycine was removed and the column was washed and equilibrated with 10 ml of PBS.

### Purification of Antibody

1 mg of anti-BSA IgG (rabbit) (anti-bovine serum albumin IgG (rabbit)) was applied to an affinity purification column, which was then rotated at room temperature for two hours. The column was washed with 15 ml of PBS. To the column was added 5 ml of 0.1 M glycine-HCl (pH 3.2) and five elutions of a volume of 1 ml each were collected in microtubes containing 100 µl of 1 M Tris. The amount of protein in the eluted fractions was quantified with a DC Protein Assay Kit (Bio-Rad). A similar adsorption-elution experiment was also carried out for human IgG.

### Results

The results are shown in Fig. 20. The column using the peptide of the present invention allowed one to recover 70 to 80% of the rabbit antibody used in the purification. This recovery rate was superior to that of the control, a Protein A column. It was also found that the affinity purification column using the peptide of the present invention was applicable to the purification of human IgG (Fig. 21). Therefore, it turned out that the means in which the peptide of the present invention was immobilized was extremely useful in antibody purification.

### Industrial Applicability

According to the present invention, a peptide capable of binding to an immunoglobulin, a fusion protein with the peptide, nucleic acids coding for the peptide and for the fusion protein, and others are obtained, and thus can be used in the fields of detection, isolation, and purification of immunoglobulins, and of pharmaceutical compositions for the treatment or prevention of diseases caused by the binding between C1q and an immunoglobulin, such as rheumatoid arthritis, and immune-complex diseases such as SLE, glomerulonephritis, vasculitis, and arthritis.

### [Sequence Listing Free Text]

SEQ ID NO:1:Immunoglobulin binding peptide
SEQ ID NO:2:Immunoglobulin binding peptide
SEQ ID NO:3:Immunoglobulin binding peptide
SEQ ID NO:4:Immunoglobulin binding peptide
SEQ ID NO:5:Immunoglobulin binding peptide
SEQ ID NO:6:Immunoglobulin binding peptide
SEQ ID NO:7:Immunoglobulin binding peptide
SEQ ID NO:23:Immunoglobulin binding peptide
SEQ ID NO:24:Immunoglobulin binding peptide
SEQ ID NO:25:Immunoglobulin binding peptide
SEQ ID NO:26:Immunoglobulin binding peptide
SEQ ID NO:27:Immunoglobulin binding peptide
SEQ ID NO:28:Immunoglobulin binding peptide
SEQ ID NO:29:Immunoglobulin binding peptide
SEQ ID NO:30:Immunoglobulin binding peptide
SEQ ID NO:31:Immunoglobulin binding peptide
SEQ ID NO:32:Immunoglobulin binding peptide

[Sequence Listing]

## Claims

1. A peptide capable of binding to an immunoglobulin, wherein the peptide is selected from the group consisting of:
(a) a peptide having the amino acid sequence depicted in any of SEQ ID NOs: 1 to 7;
(b) a peptide having an amino acid sequence in which one or more amino acids are deleted, substituted, or added in the amino acid sequence depicted in any of SEQ ID NOs: 1 to 7; and
(c) a peptide having an amino acid sequence of 66.7% or more homology to the amino acid sequence depicted in any of SEQ ID NOs: 1 to 7,
and wherein cysteine(s) in the peptide may be optionally substituted by different kind(s) of amino acid(s).

2. The peptide according to claim 1, wherein cysteine(s) in the peptide is(are) substituted by serine(s).

3. The peptide according to claim 2, which has the amino acid sequence depicted in any of SEQ ID NOs: 28 to 32.

4. A peptide capable of binding to an immunoglobulin, wherein the peptide is selected from the group consisting of:
(a) a peptide having the amino acid sequence depicted in any of SEQ ID NOs: 1 to 7;
(b) a peptide having an amino acid sequence in which one or more amino acids are deleted, substituted, or added in the amino acid sequence depicted in any of SEQ ID NOs: 1 to 7; and
(c) a peptide having an amino acid sequence of 66.7% or more homology to the amino acid sequence depicted in any of SEQ ID NOs: 1 to 7,
and wherein amino acid(s) in the peptide may be optionally substituted by the corresponding D-amino acid(s).

5. The peptide according to claim 4, wherein all amino acids are substituted by the corresponding D-amino acids.

6. The peptide according to claim 5, which has the amino acid sequence represented by dPro-Gly-dLeu-dTyr-dTyr-dPhe.

7. A peptide capable of binding to an immunoglobulin, wherein the peptide is selected from the group consisting of:
(a) a peptide having the amino acid sequence depicted in any of SEQ ID NOs: 1 to 7;
(b) a peptide having an amino acid sequence in which one or more amino acids are deleted, substituted, or added in the amino acid sequence depicted in any of SEQ ID NOs: 1 to 7; and
(c) a peptide having an amino acid sequence of 66.7% or more homology to the amino acid sequence depicted in any of SEQ ID NOs: 1 to 7,
and wherein cysteine(s) in the peptide may be optionally substituted by different kind(s) of amino acid(s), and amino acid(s) in the peptide may be optionally substituted by the corresponding D-amino acid(s).

8. The peptide according to claim 7, wherein cysteine(s) in the peptide is substituted by different kind(s) of amino acid(s), and all amino acids are substituted by the corresponding D-amino acids.

9. A nucleic acid coding for a peptide capable of binding to an immunoglobulin, wherein the nucleic acid is selected from the group consisting of:
(a) a nucleic acid coding for the peptide according to (1) ;
(b) a nucleic acid having the nucleotide sequence depicted in any of SEQ ID NOs: 33 to 37;
(c) a nucleic acid having a nucleotide sequence in which one or more nucleotides are deleted, substituted, or added in the nucleotide sequence depicted in any of SEQ ID NOs: 33 to 37;
(d) a nucleic acid hybridizable to the nucleic acid of (b) or (c) as described above or the complementary strand thereof under stringent conditions; and
(e) a nucleic acid having a nucleotide sequence of 50% or more homology to the nucleotide sequence depicted in any of SEQ ID NOs: 33 to 37.

10. The nucleic acid according to claim 9, which has the nucleotide sequence depicted in any of SEQ ID NOs: 33 to 37.

11. A vector including the nucleic acid according to claim 9 or 10.

12. A fusion protein, which has the peptide according to any of claims 1 to 3 added at the N-terminus and/or C-terminus of a desired protein.

13. A fusion protein, which has the peptide according to any of claims 4 to 8 added at the N-terminus and/or C-terminus of a desired protein.

14. A nucleic acid coding for the fusion protein according to claim 12.

15. A vector including the nucleic acid according to claim 14.

16. A cell including the nucleic acid according to claim 9 or 10, the nucleic acid according to claim 14, or the vector according to claim 11 or 15.

17. A method for producing a peptide capable of binding to an immunoglobulin, wherein the method includes the steps of:
(a) transforming a cell with the vector according to (11), and
(b) culturing the cell to produce the peptide.

18. A peptide capable of binding to an immunoglobulin, which is obtainable by the method according to claim 17.

19. A method for producing a fusion protein in which a peptide capable of binding to an immunoglobulin is added at the N-terminus and/or C-terminus of a desired protein, wherein the method comprises the steps of:
(a) transforming a cell with the vector according to claim 15, and
(b) culturing the cell to produce the fusion protein.

20. The method according to claim 19, further comprising the step of removing the desired protein from the fusion protein.

21. A fusion protein which is obtainable by the method according to claim 19 or 20.

22. A composition for.binding an immunoglobulin, which comprises the peptide according to any of claims 1 to 8 or the fusion protein according to claim 12 or 13.

23. The composition according to claim 22, which is used for determining the presence or the amount of the immunoglobulin, or for isolating the immunoglobulin.

24. A means for binding an immunoglobulin, wherein the peptide according to any of claims 1 to 8 or the fusion protein according to claim 12 or 13 is immobilized.

25. The means according to claim 24, which is used for determining the presence or the amount of the immunoglobulin, or for isolating the immunoglobulin.

26. A method for binding an immunoglobulin, comprising:
(a) adding to a sample the peptide according to any of claims 1 to 8 or the fusion protein according to claim 12 or 13, and
(b) determining a complex of the peptide or fusion protein and the immunoglobulin.

27. A kit comprising a peptide capable of binding to an immunoglobulin or a fusion protein including the peptide, for use in the method according to claim 26.

28. A pharmaceutical composition for the treatment or prevention of a disease caused by the binding between C1q and an immunoglobulin, wherein the pharmaceutical composition comprises the peptide according to any of claims 1 to 8 or the fusion protein according to 12 or 13.

29. The pharmaceutical composition according to claim 28, wherein the disease is rheumatoid arthritis.

30. The pharmaceutical composition according to claim 28, wherein the disease is an immune-complex disease, such as systemic lupus erythematosus (SLE), glomerulonephritis, vasculitis, or arthritis.

31. The peptide according to any of claims 1 to 8 or the fusion protein according to claim 12 or 13, which is labeled.

32. A method for detecting an antibody in a sample, which includes reacting the labeled peptide or fusion protein according to claim 31 with an antibody in a sample, and then detecting the peptide or fusion protein bound to the antibody.
